# EUROPEAN PATENT APPLICATION

(11) **EP 4 821 924 A1**
(43) Date of publication of application: **14.10.2026**
(21) Application number: 24899985.6
(22) Date of filing: 06.12.2024
(51) Int. Cl.: C07D 401/04, C07D 471/04, A61K 31/502, A61K 31/519, A61P 35/00

(54) **FUSED RING MAT2A INHIBITOR, AND PHARMACEUTICAL COMPOSITION CONTAINING SAME AND USE THEREOF**

(30) Priority: 08.12.2023 CN 202311682656
(71) Applicant: Euregen Biopharma Co., Ltd., Zhongshan, Guangdong 528451 (CN)
(72) Inventor: LI, Jing, Zhongshan, Guangdong 528451 (CN); CHEN, Yanhong, Zhongshan, Guangdong 528451 (CN); DENG, Wenjia, Zhongshan, Guangdong 528451 (CN); CHEN, Xuxing, Zhongshan, Guangdong 528451 (CN)
(74) Representative: dompatent
(86) International application number: PCT/CN2024/137538
(87) International publication number: WO 2025/119352

(57) **Abstract**

Provided are a fused ring MAT2A inhibitor, and a pharmaceutical composition containing same and a use thereof. Specifically, provided are a compound represented by formula I, or a pharmaceutically acceptable salt, enantiomer, diastereomer, tautomer, cis-trans isomer, solvate, polymorphic substance, and deuterated substance thereof, or a combination thereof, wherein each variable is as defined herein, and a use of the compound and a pharmaceutical composition containing the compound in the preparation of a drug for preventing or treating MTAP-/--related cancers.

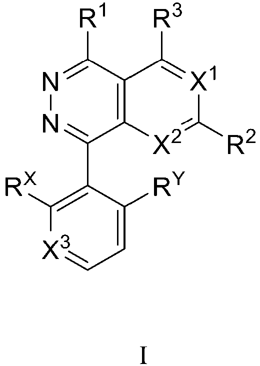

## Description

### TECHNICAL FIELD

The present invention pertains to the field of medicinal chemistry. More specifically, the present invention relates to a fused bicyclic MAT2A inhibitor, a process for the preparation thereof, a pharmaceutical composition comprising the same, and uses thereof.

### BACKGROUND

Cancer is one of the leading causes of death worldwide. One limitation of commonly used therapies, such as chemotherapy and immunotherapy, is that their cytotoxicity is not restricted to cancer cells, and adverse effects may occur in normal tissues. Accordingly, new strategies are needed to better target cancer cells.

The phenomenon in which cell death occurs when the expression of two or more genes is simultaneously deficient, while a deficiency in the expression of only one of them does not result in cell death, is referred to as synthetic lethality. The concept of synthetic lethality originates from studies of Drosophila models, in which it was found that flies bearing two specific gene mutations simultaneously could not survive, whereas a mutation in either gene alone did not cause lethal injury to the flies. Accordingly, certain tumors with specific mutations can be treated through a synthetic lethality strategy: that is, compared with normal tissues, certain tumors harbor gene defects, and by artificially inhibiting another signaling pathway, the tumor tissue becomes more vulnerable while normal tissues are largely unaffected, thereby achieving the goal of selectively killing the tumor. The PARP1 inhibitor is recognized as the first successful anticancer drug developed using the synthetic lethality concept. Because tumor suppressor gene defects account for the vast majority of tumor-specific mutations, the synthetic lethality strategy is expected to lead to additional precision anti-tumor drugs.

Methionine adenosyltransferase 2A (MAT2A) is a key enzyme that produces S-adenosyl methionine (SAM) in cells, catalyzing the synthesis of SAM from methionine (Met) and adenosine triphosphate (ATP). SAM is a major methyl donor in cells; under the catalysis of various methyltransferases, the methyl group can be transferred from SAM to a variety of substrates, including DNA, RNA, and proteins. Protein arginine N-methyltransferase 5 (PRMT5) is one such methyltransferase. Because methylthioadenosine (MTA) is structurally similar to SAM, MTA can compete with SAM and inhibit the activity of PRMT5. MTA can be converted by an enzyme called methylthioadenosine phosphorylase (MTAP) into adenine and 5-methylthioribose-1-phosphate, and therefore the concentration of MTA in normal cells can be maintained at a low level. Because the MTAP gene is located adjacent to the tumor suppressor gene p16/CDKN2A, it is co-deleted with p16/CDKN2A in approximately 15% of human cancers. Solid tumors with a relatively high frequency of MTAP deletion include glioma, mesothelioma, esophageal cancer, bladder cancer, pancreatic cancer, melanoma, non-small cell lung cancer, head and neck cancer, sarcoma, and cholangiocarcinoma. In these co-deleted tumor cells, loss of MTAP results in the accumulation of MTA, and the abnormally elevated MTA inhibits PRMT5 in the tumor tissue; therefore, MTAP-deleted tumor cells are more dependent on the production of SAM, and inhibiting MAT2A to reduce SAM levels can selectively kill MTAP-deleted tumor cells. MAT2A gene knockout was performed in approximately 400 cancer cell lines, and it was found that, compared with MTAP WT cells, a greater proportion of MTAP-deleted cells lost viability. In addition, knockdown of MAT2A protein can reduce tumor growth in vivo. These results suggest that MAT2A inhibitors may provide a new therapeutic approach for cancer patients with MTAP deletion.

IDE397 is a MAT2A inhibitor at the forefront of clinical development. Preclinical studies have shown that this compound exhibits significant efficacy in non-small cell lung cancer, bladder cancer, pancreatic cancer, head and neck cancer, gastric cancer, and esophageal cancer models (AACR 2021). Phase II clinical trials further demonstrated that the compound is effective in patients with non-small cell lung cancer and urothelial carcinoma.

Therefore, there is an urgent need to develop a compound having a good MAT2A inhibitory effect, for use in the preparation of a medicament for treating MTAP^{-/-} associated cancers.

### SUMMARY OF THE INVENTION

An object of the present invention is to provide a fused bicyclic compound having a novel structure and a good MAT2A inhibitory effect, as well as a pharmaceutical composition comprising the same and uses thereof.

In a first aspect of the present invention, there is provided a compound of Formula I, or a pharmaceutically acceptable salt, enantiomer, diastereomer, tautomer, cis-trans isomer, solvate, polymorph, deuterated form thereof, or a combination thereof, 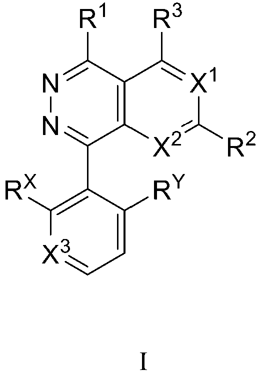 wherein:
R¹ is selected from the group consisting of: hydrogen, hydroxy, optionally substituted C₁-C₆ alkyl, optionally substituted C₂-C₆ alkenyl, optionally substituted C₂-C₆ alkynyl, optionally substituted 3- to 10-membered carbocyclyl, optionally substituted 4- to 8-membered heterocyclyl, optionally substituted 6- to 10-membered aryl, optionally substituted 5- to 10-membered heteroaryl, -L¹-R⁴, and -NR⁵R⁶; in R¹, said "optionally substituted" means that the specified group is unsubstituted or substituted by one or more R^{1s}, and each R^{1s} is independently selected from the group consisting of: halo, hydroxy, amino, cyano, oxo, C₁-C₆ alkyl, C₁-C₆ haloalkyl, C₂-C₄ alkenyl, C₂-C₄ haloalkenyl, C₂-C₄ alkynyl, C₂-C₄ haloalkynyl, C₁-C₆ alkoxy-C₁-C₆ alkyl, hydroxy C₁-C₆ alkyl- (i.e., C₁-C₆ hydroxyalkyl), C₁-C₆ alkylamino, C₁-C₆ alkoxy, C₁-C₆ haloalkoxy, 3- to 10-membered carbocyclyl, 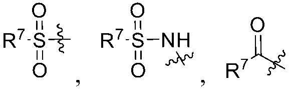 and 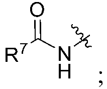
L¹ is selected from the group consisting of: -O-, -S-, -SO-, -SO₂-, and -CO-;
R⁴ is selected from the group consisting of: optionally substituted C₁-C₆ alkyl, optionally substituted 3- to 10-membered carbocyclyl, optionally substituted 4- to 8-membered heterocyclyl, optionally substituted 6- to 10-membered aryl, and optionally substituted 5- to 10-membered heteroaryl; in R⁴, said "optionally substituted" means that the specified group is unsubstituted or substituted by one or more R^{4s}, and each R^{4s} is independently selected from the group consisting of: halo, hydroxy, amino, cyano, oxo, C₁-C₆ alkoxy, C₁-C₆ haloalkoxy, C₂-C₄ alkenyl, C₂-C₄ haloalkenyl, C₂-C₄ alkynyl, C₂-C₄ haloalkynyl, C₁-C₆ alkoxy-C₁-C₆ alkyl, hydroxy C₁-C₆ alkyl, amino C₁-C₆ alkyl, 3- to 10-membered carbocyclyl, 3- to 10-membered carbocyclyloxy, 4- to 8-membered heterocyclyl, 4- to 8-membered heterocyclyloxy, 6- to 10-membered aryl, 5- to 10-membered heteroaryl, 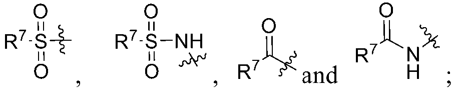
R⁵ is selected from the group consisting of: hydrogen, C₁-C₆ alkyl, C₁-C₆ haloalkyl, and 3- to 10-membered carbocyclyl;
R⁶ is selected from the group consisting of: hydrogen, optionally substituted C₁-C₆ alkyl, optionally substituted 3- to 10-membered carbocyclyl, optionally substituted 4- to 10-membered heterocyclyl, optionally substituted 6- to 10-membered aryl, optionally substituted 5- to 10-membered heteroaryl, 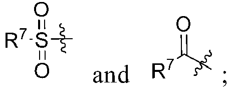 in R⁶, said "optionally substituted" means that the specified group is unsubstituted or substituted by one or more R^{6s}, and each R^{6s} is independently selected from the group consisting of: deuterium, halo, hydroxy, amino, cyano, C₁-C₆ alkyl, C₁-C₆ haloalkyl, hydroxy C₁-C₆ alkyl, C₁-C₆ alkoxy, C₁-C₆ haloalkoxy, C₂-C₄ alkenyl, C₂-C₄ haloalkenyl, C₂-C₄ alkynyl, C₂-C₄ haloalkynyl, 3- to 10-membered carbocyclyl optionally substituted by one or more R⁸, 3- to 10-membered carbocyclyloxy optionally substituted by one or more R⁸, 4- to 8-membered heterocyclyl optionally substituted by one or more R⁸, 4- to 8-membered heterocyclyloxy optionally substituted by one or more R⁸, C₁-C₆ alkylamino, (C₁-C₆ alkyl)₂ amino, C₁-C₆ alkoxy-C₁-C₆ alkyl, amino C₁-C₆ alkyl, 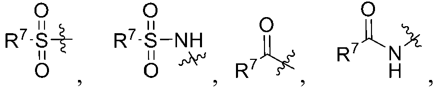 6- to 10-membered aryl optionally substituted by one or more R⁹, and 5- to 10-membered heteroaryl optionally substituted by one or more R⁹;
R² is selected from: halo, cyano, optionally substituted C₁-C₆ alkyl, optionally substituted C₂-C₆ alkenyl, optionally substituted C₂-C₆ alkynyl, optionally substituted 3- to 10-membered carbocyclyl, optionally substituted 4- to 8-membered heterocyclyl, -L²-R¹⁰, and -NR¹¹R¹²; in R², said "optionally substituted" means that the specified group is unsubstituted or substituted by one or more R^{2s}, and each R^{2s} is independently selected from the group consisting of: halo, cyano, oxo, C₁-C₆ alkyl, C₁-C₆ haloalkyl, C₁-C₆ alkoxy, C₁-C₆ haloalkoxy, and 3- to 10-membered carbocyclyl;
R³ is selected from the group consisting of: hydrogen, halo, cyano, optionally substituted C₁-C₆ alkyl, optionally substituted C₂-C₆ alkenyl, optionally substituted C₂-C₆ alkynyl, optionally substituted 3- to 10-membered carbocyclyl, optionally substituted 4- to 8-membered heterocyclyl, -L^{2'}-R^{10'}, and -NR^{11'}R^{12'}; in R³, said "optionally substituted" means that the specified group is unsubstituted or substituted by one or more R^{3s}, and each R^{3s} is independently selected from the group consisting of: halo, cyano, oxo, C₁-C₆ alkyl, C₁-C₆ haloalkyl, C₁-C₆ alkoxy, C₁-C₆ haloalkoxy, and 3- to 10-membered carbocyclyl;
L² and L^{2'} are each independently selected from the group consisting of: -O-, -S-, -SO-, -SO₂-, and -CO-;
R¹⁰ and R^{10'} are each independently selected from the group consisting of: optionally substituted C₁-C₆ alkyl, optionally substituted 3- to 10-membered carbocyclyl, optionally substituted 4- to 8-membered heterocyclyl, optionally substituted 6- to 10-membered aryl, and optionally substituted 5- to 10-membered heteroaryl; in R¹⁰ and R^{10'}, said "optionally substituted" means that the specified group is unsubstituted or substituted by one or more R^{10s}, and each R^{10s} is independently selected from the group consisting of: halo, cyano, C₁-C₆ alkyl, C₁-C₆ haloalkyl, C₁-C₆ alkoxy, C₁-C₆ haloalkoxy, 3- to 10-membered carbocyclyl, 4- to 8-membered heterocyclyl, 6- to 10-membered aryl, and 5- to 10-membered heteroaryl;
R¹¹ and R^{11'} are each independently selected from the group consisting of: hydrogen and C₁-C₆ alkyl;
R¹² and R^{12'} are each independently selected from the group consisting of: optionally substituted C₁-C₆ alkyl, optionally substituted 3- to 10-membered carbocyclyl, optionally substituted 4- to 10-membered heterocyclyl, optionally substituted 6- to 10-membered aryl, optionally substituted 5- to 10-membered heteroaryl, 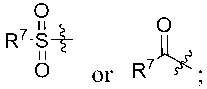 in R¹² and R^{12'}, said "optionally substituted" means that the specified group is unsubstituted or substituted by one or more R^{12s}, and each R^{12s} is independently selected from the group consisting of: halo, cyano, C₁-C₆ alkyl, C₁-C₆ haloalkyl, C₁-C₆ alkoxy, C₁-C₆ haloalkoxy, 3- to 10-membered carbocyclyl, and 4- to 8-membered heterocyclyl;
X³ is selected from the group consisting of: CH and N;
R^{X} is selected from the group consisting of: methyl, ethyl, 3- to 4-membered carbocyclyl, halomethyl, or halo;
R^{Y} is selected from the group consisting of: hydrogen, methyl, halomethyl, or halo;
X¹ is selected from the group consisting of: CR¹³ and N;
X² is selected from the group consisting of: CR¹⁴ and N;
R¹³ and R¹⁴ are each independently selected from the group consisting of: hydrogen, halo, cyano, optionally substituted C₁-C₆ alkyl, optionally substituted C₁-C₆ alkoxy, optionally substituted 3- to 10-membered carbocyclyl, and optionally substituted 4- to 8-membered heterocyclyl; in R¹³ and R¹⁴, said "optionally substituted" means that the specified group is unsubstituted or substituted by one or more R^{13s} or R^{14s}, and each R^{13s} and R^{14s} is independently selected from the group consisting of: halo and oxo; with the proviso that, when R^{X} and R² are simultaneously chloro and X¹ is CR¹³, R¹³ is not chloro;
each R⁷ is independently selected from the group consisting of: C₁-C₆ alkyl, C₁-C₆ haloalkyl, C₁-C₆ alkoxy, C₁-C₆ haloalkoxy, 3- to 10-membered carbocyclyl, 4- to 8-membered heterocyclyl, amino, C₁-C₆ alkylamino (i.e., -NH(C₁-C₆ alkyl)), di(C₁-C₆ alkyl)amino (i.e., -N(C₁-C₆ alkyl)₂), 6- to 10-membered aryl, and 5- to 10-membered heteroaryl;
each R⁸ is independently selected from the group consisting of: hydrogen, halo, hydroxy, C₁-C₆ alkyl, C₁-C₆ haloalkyl, or hydroxy C₁-C₆ alkyl;
each R⁹ is independently selected from the group consisting of: hydrogen, halo, C₁-C₆ alkyl, or C₁-C₆ haloalkyl.

In another preferred embodiment, R¹ is selected from the group consisting of: hydrogen, hydroxy, optionally substituted C₁-C₆ alkyl, optionally substituted 3- to 10-membered carbocyclyl, optionally substituted 4- to 8-membered heterocyclyl, optionally substituted 5- to 10-membered heteroaryl, -OR⁴, and -NR⁵R⁶; preferably, R¹ is selected from the group consisting of: hydrogen, hydroxy, optionally substituted 4- to 8-membered heterocyclyl, or -NR⁵R⁶.

In another preferred embodiment, R¹ is selected from the group consisting of: hydrogen, optionally substituted C₁-C₆ alkyl (preferably, C₁-C₆ alkyl), and -NR⁵R⁶.
In another preferred embodiment, R⁵ is selected from the group consisting of: hydrogen and C₁-C₆ alkyl; preferably, R⁵ is hydrogen.

In another preferred embodiment, R⁶ is selected from the group consisting of: optionally substituted C₁-C₆ alkyl, optionally substituted 3- to 10-membered carbocyclyl, and optionally substituted 5- to 10-membered heteroaryl; preferably, R⁶ is selected from the group consisting of: hydrogen, optionally substituted C₁-C₆ alkyl.

In another preferred embodiment, each R^{6s} is independently selected from the group consisting of: deuterium, halo, C₁-C₃ alkyl, C₁-C₃ haloalkyl, C₂-C₄ alkynyl, C₁-C₃ alkoxy, C₁-C₃ haloalkoxy, and 6- to 10-membered aryl; preferably, each R^{6s} is independently selected from the group consisting of: deuterium and halo.

In another preferred embodiment, R³ is hydrogen, and R⁶ is selected from the group consisting of: hydrogen, optionally substituted C₁-C₆ alkyl; and R^{6s} is deuterium.

In another preferred embodiment, R² is selected from the group consisting of: halo, optionally substituted C₁-C₆ alkyl, optionally substituted 3- to 10-membered carbocyclyl, optionally substituted 4- to 8-membered heterocyclyl, and -L²-R¹⁰.

In another preferred embodiment, each R^{2s} is independently selected from the group consisting of: halo, cyano, oxo, C₁-C₆ alkyl, C₁-C₆ haloalkyl, C₁-C₆ alkoxy, C₁-C₆ haloalkoxy, and 3- to 8-membered carbocyclyl; preferably, each R^{2s} is independently selected from the group consisting of: halo, C₁-C₆ alkyl.

In another preferred embodiment, R³ is selected from the group consisting of: hydrogen, halo, optionally substituted C₁-C₆ alkyl, optionally substituted C₂-C₆ alkenyl, optionally substituted C₂-C₆ alkynyl, optionally substituted 3- to 10-membered carbocyclyl, optionally substituted 4- to 8-membered heterocyclyl, -L^{2'}-R^{10'}; preferably, R³ is selected from the group consisting of: hydrogen, optionally substituted C₁-C₆ alkyl.

In another preferred embodiment, each R^{3s} is independently selected from the group consisting of: halo, cyano, oxo, C₁-C₆ alkyl, C₁-C₆ haloalkyl, C₁-C₆ alkoxy, C₁-C₆ haloalkoxy, and 3- to 8-membered carbocyclyl.

In another preferred embodiment, R¹⁰ and R^{10'} are each independently selected from the group consisting of: optionally substituted C₁-C₆ alkyl, optionally substituted 3- to 8-membered carbocyclyl, optionally substituted 4- to 8-membered heterocyclyl.

In another preferred embodiment, each R^{10s} is independently selected from the group consisting of: halo, cyano, C₁-C₆ alkyl, C₁-C₆ haloalkyl, C₁-C₆ alkoxy, C₁-C₆ haloalkoxy, 3- to 8-membered carbocyclyl, 4- to 8-membered heterocyclyl.

In another preferred embodiment, R² is selected from the group consisting of: halo (e.g., F, Cl, Br), optionally substituted C₁-C₆ alkyl (preferably, C₁-C₆ alkyl or C₁-C₆ haloalkyl; more preferably, C₁-C₆ haloalkyl, e.g., trifluoromethyl), and optionally substituted 3- to 10-membered carbocyclyl (preferably, 3- to 10-membered carbocyclyl; more preferably, C₃-C₆ cycloalkyl; most preferably, selected from cyclopropyl, cyclobutyl).

In another preferred embodiment, R³ is -L^{2'}-R^{10'}; wherein L^{2'} is -O- and R^{10'} is optionally substituted C₁-C₆ alkyl (preferably, R^{10'} is C₁-C₆ alkyl; more preferably, R^{10'} is methyl).

In another preferred embodiment, R³ is hydrogen.

In another preferred embodiment, R³ is hydrogen or -L^{2'}-R^{10'}; wherein L^{2'} is -O- and R^{10'} is methyl (i.e., R³ is hydrogen or methoxy).

In another preferred embodiment, R¹³ and R¹⁴ are each independently selected from the group consisting of: hydrogen, halo, cyano, C₁-C₄ alkyl, or C₁-C₄ haloalkyl.

In another preferred embodiment, R¹³ is hydrogen or halo (e.g., F).

In another preferred embodiment, X¹ is CR¹³; preferably, CH or CF.

In another preferred embodiment, X¹ is CR¹³; preferably, CH.

In another preferred embodiment, X² is CR¹⁴; preferably, X² is CR¹⁴ wherein R¹⁴ is selected from the group consisting of: hydrogen, halo (e.g., F), C₁-C₄ alkyl (e.g., methyl), and optionally substituted 3- to 10-membered carbocyclyl (preferably, 3- to 4-membered carbocyclyl; more preferably, 3- to 4-membered saturated carbocyclyl, e.g., cyclopropyl).

In another preferred embodiment, X² is CR¹⁴; preferably, X² is CR¹⁴ wherein R¹⁴ is selected from the group consisting of: hydrogen, halo (e.g., F), C₁-C₄ alkyl (e.g., methyl).

In another preferred embodiment, X³ is N.

In another preferred embodiment, in R^{X}, said 3- to 4-membered carbocyclyl is a 3- to 4-membered saturated carbocyclyl; preferably, cyclopropyl.

In another preferred embodiment, R^{X} is selected from the group consisting of: methyl, ethyl, halomethyl, or halo.

In another preferred embodiment, R^{X} is methyl, halo, or cyclopropyl.

In another preferred embodiment, R^{X} is methyl or halo.

In another preferred embodiment, R^{Y} is hydrogen, methyl, or halo (e.g., F).

In another preferred embodiment,
R¹ is selected from the group consisting of: hydrogen, hydroxy, optionally substituted C₁-C₆ alkyl, optionally substituted 3- to 10-membered carbocyclyl, optionally substituted 4- to 8-membered heterocyclyl, optionally substituted 5- to 10-membered heteroaryl, -OR⁴, and -NR⁵R⁶; in R¹, said "optionally substituted" means that the specified group is unsubstituted or substituted by one or more R^{1s}, and each R^{1s} is independently selected from the group consisting of: halo, hydroxy, amino, cyano, oxo, C₁-C₆ alkyl, C₁-C₆ haloalkyl, C₁-C₆ alkoxy-C₁-C₆ alkyl, hydroxy C₁-C₆ alkyl, C₁-C₆ alkylamino, C₁-C₆ alkoxy, and C₁-C₆ haloalkoxy.

In another preferred embodiment, R¹ is selected from the group consisting of: hydrogen, hydroxy, optionally substituted 4- to 8-membered heterocyclyl, or -NR⁵R⁶; wherein said heterocyclyl is optionally substituted by a substituent selected from the group consisting of: halo, C₁-C₃ alkyl, C₁-C₃ haloalkyl, C₁-C₃ alkoxy, C₁-C₃ haloalkoxy, hydroxy, and amino;
R⁵ is selected from the group consisting of: hydrogen and C₁-C₆ alkyl;
R⁶ is selected from the group consisting of: optionally substituted C₁-C₆ alkyl, optionally substituted 3- to 10-membered carbocyclyl, and optionally substituted 5- to 10-membered heteroaryl; in R⁶, said "optionally substituted" means that the specified group is unsubstituted or substituted by one or more R^{6s}, and each R^{6s} is independently selected from the group consisting of: deuterium, halo, C₁-C₃ alkyl, C₁-C₃ haloalkyl, C₂-C₄ alkynyl, C₁-C₃ alkoxy, C₁-C₃ haloalkoxy, and 6- to 10-membered aryl.

In another preferred embodiment, R¹ is selected from the group consisting of: hydrogen, optionally substituted C₁-C₆ alkyl, optionally substituted 4- to 8-membered heterocyclyl, or -NR⁵R⁶; wherein R⁵ is hydrogen, and R⁶ is selected from the group consisting of: hydrogen, optionally substituted C₁-C₆ alkyl, and optionally substituted 5- to 10-membered heteroaryl; preferably, in R⁶, said "optionally substituted" means that the specified group is unsubstituted or substituted by one or more R^{6s}, and R^{6s} is deuterium or C₁-C₆ alkyl.

In another preferred embodiment, in R¹, the 5- to 10-membered heteroaryl is a 9- to 10-membered heteroaryl.

In another preferred embodiment, in R¹, said heteroaryl contains only 1, 2, 3, or 4 N as heteroatoms.

In another preferred embodiment, R¹ is selected from the group consisting of: hydrogen, optionally substituted C₁-C₆ alkyl, and -NR⁵R⁶; wherein R⁵ is hydrogen, and R⁶ is selected from the group consisting of: hydrogen, optionally substituted C₁-C₆ alkyl.

In another preferred embodiment,
R² is selected from the group consisting of: halo, optionally substituted C₁-C₆ alkyl, optionally substituted 3- to 10-membered carbocyclyl, optionally substituted 4- to 8-membered heterocyclyl, and -L²-R¹⁰; in R², said "optionally substituted" means that the specified group is unsubstituted or substituted by one or more R^{2s}, and each R^{2s} is independently selected from the group consisting of: halo, cyano, oxo, C₁-C₆ alkyl, C₁-C₆ haloalkyl, C₁-C₆ alkoxy, C₁-C₆ haloalkoxy, and 3- to 8-membered carbocyclyl;
R³ is selected from the group consisting of: hydrogen, halo, optionally substituted C₁-C₆ alkyl, optionally substituted C₂-C₆ alkenyl, optionally substituted C₂-C₆ alkynyl, optionally substituted 3- to 10-membered carbocyclyl, optionally substituted 4- to 8-membered heterocyclyl, -L^{2'}-R^{10'}; in R³, said "optionally substituted" means that the specified group is unsubstituted or substituted by one or more R^{3s}, and each R^{3s} is independently selected from the group consisting of: halo, cyano, oxo, C₁-C₆ alkyl, C₁-C₆ haloalkyl, C₁-C₆ alkoxy, C₁-C₆ haloalkoxy, and 3- to 8-membered carbocyclyl;
R¹⁰ and R^{10'} are each independently selected from the group consisting of: optionally substituted C₁-C₆ alkyl, optionally substituted 3- to 8-membered carbocyclyl, optionally substituted 4- to 8-membered heterocyclyl; in R¹⁰ and R^{10'}, said "optionally substituted" means that the specified group is unsubstituted or substituted by one or more R^{10s}, and each R^{10s} is independently selected from the group consisting of: halo, cyano, C₁-C₆ alkyl, C₁-C₆ haloalkyl, C₁-C₆ alkoxy, C₁-C₆ haloalkoxy, 3- to 8-membered carbocyclyl, 4- to 8-membered heterocyclyl.

In another preferred embodiment, R² is selected from the group consisting of: halo, optionally substituted C₁-C₆ alkyl (preferably, C₁-C₆ haloalkyl), and optionally substituted 3-to 10-membered carbocyclyl. In another preferred embodiment, the 3- to 10-membered carbocyclyl is a 3- to 10-membered saturated carbocyclyl; preferably, a 3- to 4-membered saturated carbocyclyl. In another preferred embodiment, each R^{2s} is independently selected from the group consisting of: halo, C₁-C₆ alkyl.

In another preferred embodiment, R^{X} is selected from the group consisting of: methyl, ethyl, halomethyl, and halo; X¹ is selected from the group consisting of: CR¹³ and N; X² is selected from the group consisting of: CR¹⁴ and N; and R¹³ and R¹⁴ are each independently selected from the group consisting of: hydrogen, halo, cyano, C₁-C₄ alkyl, or C₁-C₄ haloalkyl.

In another preferred embodiment, the proviso is that when R^{X} and R² are simultaneously chloro and X¹ is CR¹³, R¹³ is not chloro.

In another preferred embodiment, X³ is selected from the group consisting of: CH and N; R^{X} is methyl or halo; R^{Y} is hydrogen, methyl, or halo.

In another preferred embodiment, R¹, R², R³, R^{X}, R^{Y}, X¹, and X² are each independently the corresponding group of the specific compounds shown in Table A and the Examples.

In another preferred embodiment, the compound is selected from the following Table A:

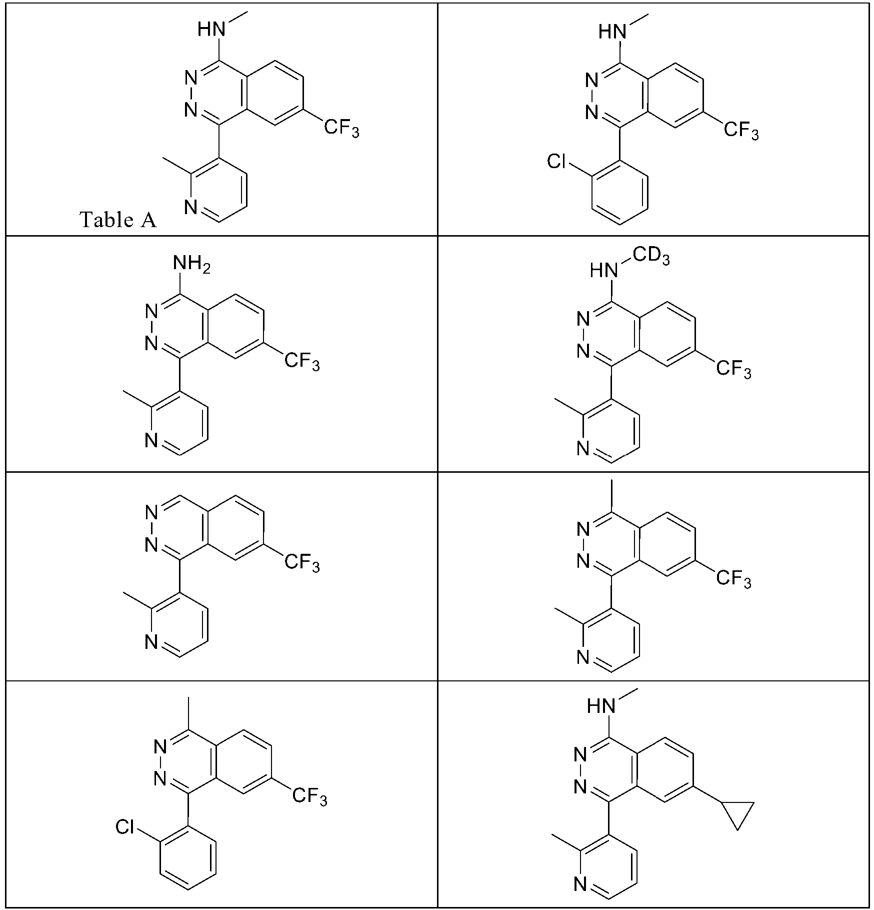

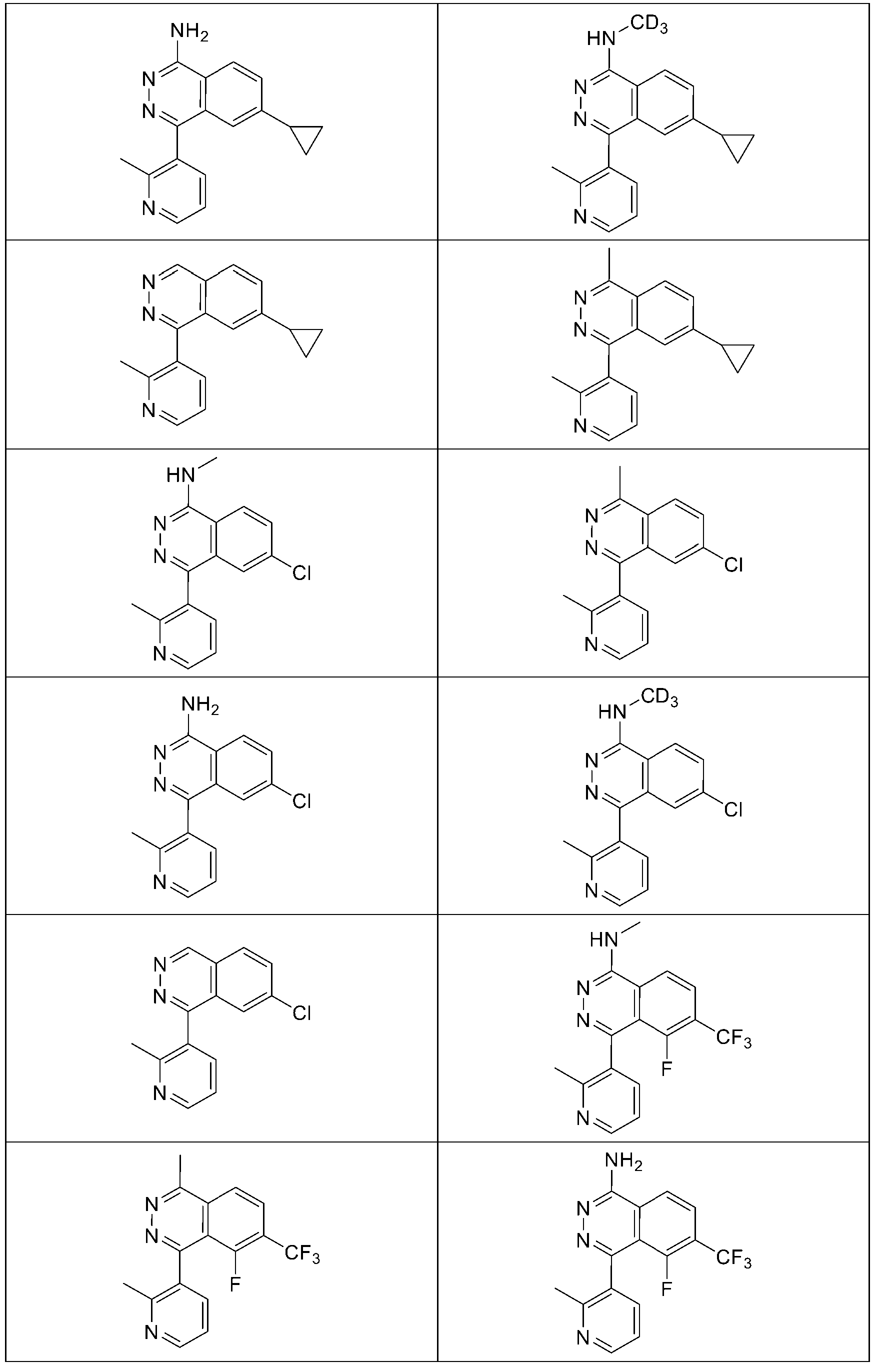

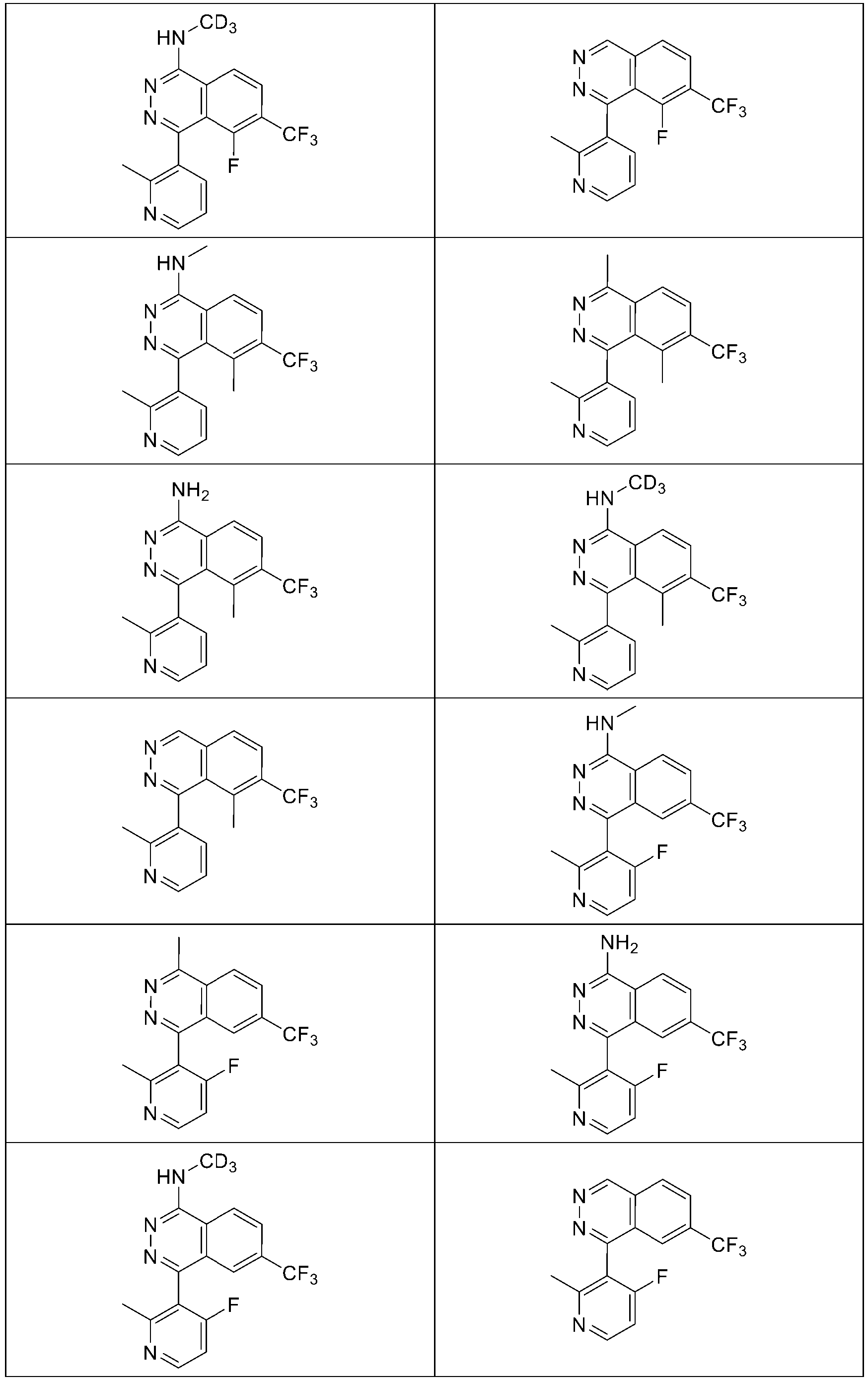

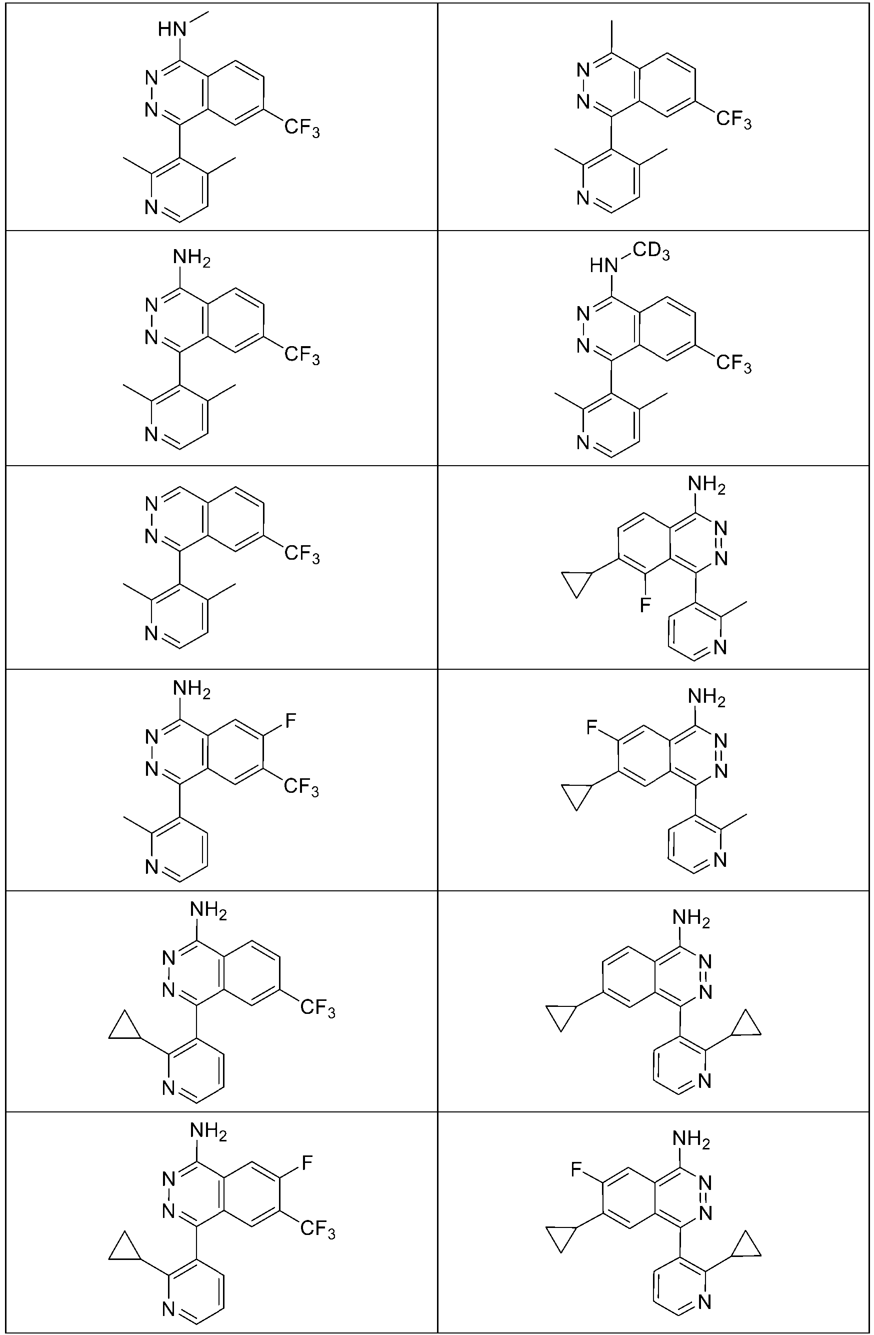

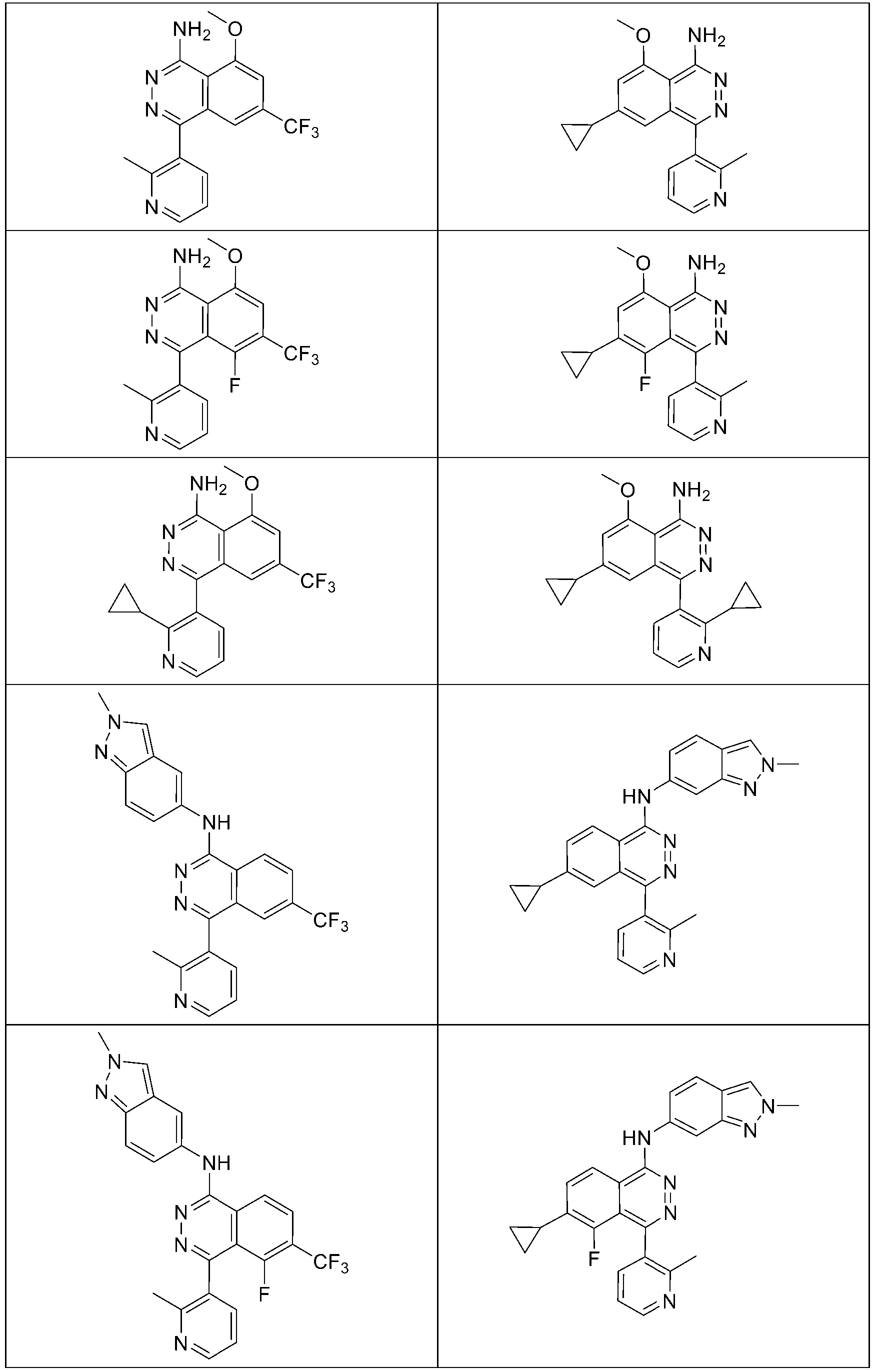

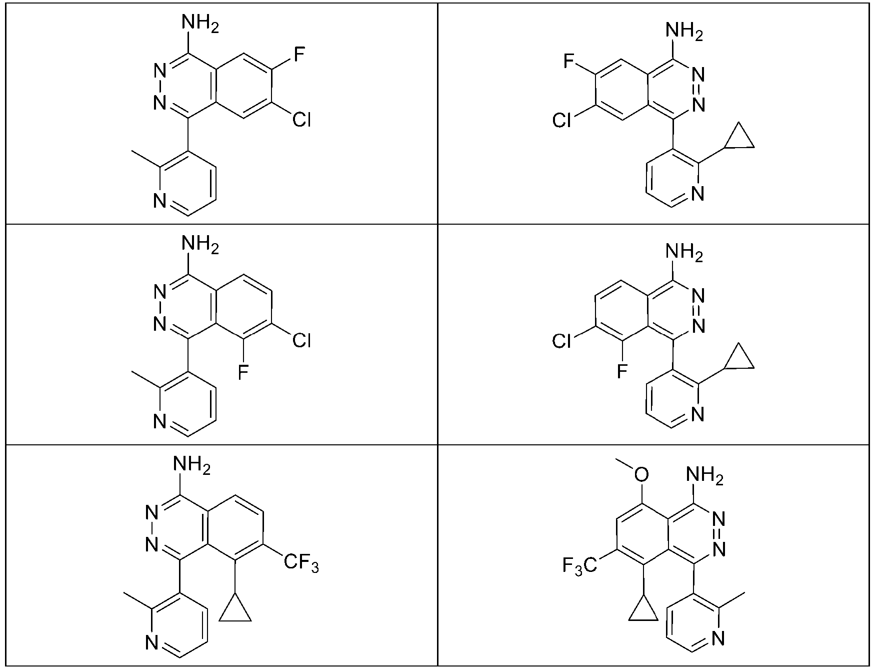

In a second aspect of the present invention, there is provided a pharmaceutical composition, said pharmaceutical composition comprising:
(1) a therapeutically effective amount of the compound according to the first aspect, or one or more of a pharmaceutically acceptable salt, enantiomer, diastereomer, tautomer, cis-trans isomer, solvate, polymorph, and deuterated form thereof as the active ingredient; and
(2) optionally, a pharmaceutically acceptable carrier.

In a third aspect of the present invention, there is provided a use of the compound according to the first aspect, or a pharmaceutically acceptable salt, enantiomer, diastereomer, tautomer, cis-trans isomer, solvate, polymorph, or deuterated form thereof, or the pharmaceutical composition according to the second aspect, in the preparation of a medicament for preventing or treating MTAP^{-/-} associated cancers.

In another preferred embodiment, said cancer is selected from: glioma, mesothelioma, esophageal cancer, bladder cancer, urothelial carcinoma, pancreatic cancer, melanoma, non-small cell lung cancer, head and neck cancer, sarcoma, cholangiocarcinoma, liver cancer, breast cancer, skin cancer, intestinal cancer, gastric cancer, renal cancer, urethral cancer, prostate cancer, testicular cancer, uterine cancer, ovarian cancer, vaginal cancer, fallopian tube cancer, multiple myeloma, spinal cord neurofibroma, astrocytoma, acute lymphoblastic leukemia, chronic lymphocytic leukemia, Hodgkin lymphoma, and non-Hodgkin lymphoma.

In another preferred embodiment, said MTAP^{-/-} associated cancer is an MTAP-deleted cancer.

In a fourth aspect of the present invention, there is provided a compound according to the first aspect, or a pharmaceutical composition according to the second aspect, for use in preventing or treating MTAP^{-/-} associated cancers.

In another preferred embodiment, said cancer is selected from: glioma, mesothelioma, esophageal cancer, bladder cancer, urothelial carcinoma, pancreatic cancer, melanoma, non-small cell lung cancer, head and neck cancer, sarcoma, cholangiocarcinoma, liver cancer, breast cancer, skin cancer, intestinal cancer, gastric cancer, renal cancer, urethral cancer, prostate cancer, testicular cancer, uterine cancer, ovarian cancer, vaginal cancer, fallopian tube cancer, multiple myeloma, spinal cord neurofibroma, astrocytoma, acute lymphoblastic leukemia, chronic lymphocytic leukemia, Hodgkin lymphoma, and non-Hodgkin lymphoma.

In another preferred embodiment, said MTAP^{-/-} associated cancer is an MTAP-deleted cancer.

In a fifth aspect of the present invention, there is provided a method for treating or preventing MTAP^{-/-} associated cancers, comprising the step of: administering to a subject in need thereof a therapeutically effective amount of the compound according to the first aspect, or a pharmaceutically acceptable salt, enantiomer, diastereomer, tautomer, cis-trans isomer, solvate, polymorph, or deuterated form thereof, or the pharmaceutical composition according to the second aspect, thereby treating or preventing MTAP^{-/-} associated cancers.

In another preferred embodiment, said cancer is as defined above.

In another preferred embodiment, said MTAP^{-/-} associated cancer is as defined above.

In a sixth aspect of the present invention, there is provided a method for inhibiting cellular proliferative activity, comprising the step of:
In another preferred embodiment, said method is in vitro and non-therapeutic.

In another preferred embodiment, said cell is a cancer cell.

In another preferred embodiment, said cell is an MTAP^{-/-} cell (i.e., an MTAP-deleted cell).

In another preferred embodiment, said cell is selected from the group consisting of: HCT116 MTAP^{-/-} cells, HCC15, NCI-H2052, LN18, NCI-H838, LU99, DOHH2, HT-1080, MKN45, THP-1, MCF7, MIA PaCa-2, U87-MG, UMUC3, or combinations thereof.

It is to be understood that, within the scope of the present invention, the above-described technical features of the present invention and the various technical features specifically described hereinafter (e.g., in the Examples) may be combined with one another to form new or preferred technical solutions. For the sake of brevity, such combinations are not enumerated individually herein.

### DETAILED DESCRIPTION

After extensive and in-depth research, the present inventors have, for the first time, synthesized a series of fused bicyclic compounds of Formula I having a novel structure, and have conducted biological activity testing thereon, unexpectedly discovering that compounds of this structural class possess favorable inhibitory effects on MAT2A. Accordingly, they can be used in the preparation of medicaments for treating MTAP^{-/-} associated cancers. The present invention has been accomplished on this basis.

### Term Definitions

Unless otherwise defined, all technical and scientific terms used herein have the same meaning as commonly understood by one of ordinary skill in the art to which this invention pertains.

As used herein, the terms "contain", "include", or "comprise" may be open-ended, semi-closed, or closed. In other words, said terms also encompass "consisting essentially of" or "consisting of".

### Group Definitions

Definitions of standard chemical terms may be found in references including Carey and Sundberg, "ADVANCED ORGANIC CHEMISTRY 4TH ED.", Vols. A (2000) and B (2001), Plenum Press, New York. Unless otherwise specified, conventional methods within the technical scope of the art are employed, such as mass spectrometry, NMR, IR and UV/VIS spectroscopy, and pharmacological methods. Unless specific definitions are provided, the terms employed herein in connection with the descriptions of analytical chemistry, organic synthetic chemistry, and pharmaceutical and medicinal chemistry are those known in the art. Standard techniques may be used in chemical synthesis, chemical analysis, pharmaceutical preparation, formulation and delivery, and treatment of patients. For example, reactions and purifications may be carried out using the manufacturer's instructions for kits, or in a manner well known in the art, or as described in the present invention. The above-described techniques and methods may generally be carried out according to conventional methods well known in the art, as described in various general and more specific references cited and discussed in this specification. In this specification, groups and substituents thereof may be selected by one of ordinary skill in the art so as to provide stable structural moieties and compounds.

When a substituent is described by a conventional chemical formula written from left to right, said substituent likewise encompasses the chemically equivalent substituent obtained when the structural formula is written from right to left. For example, -CH2O- is equivalent to -OCH2-.

The section headings used herein are for organizational purposes only and shall not be construed as limiting the subject matter described. All references or portions of references cited in this application, including but not limited to patents, patent applications, articles, books, operation manuals, and treatises, are hereby incorporated by reference in their entirety.

For certain chemical groups defined herein, a simplified notation is used as a prefix to indicate the total number of carbon atoms present in the group. For example, C₁-C₆ alkyl refers to an alkyl group as defined hereinafter having a total of 1 to 6 carbon atoms. The total number of carbon atoms in the simplified notation does not include carbon atoms that may be present in substituents of said group.

In addition to the foregoing, when used in the specification and claims of the present application, unless otherwise specifically indicated, the following terms shall have the meanings set forth below.

In the present application, the term "halo" refers to fluoro, chloro, bromo, or iodo.

"hydroxy" refers to the -OH group.

"Hydroxyalkyl" or "hydroxyalkyl group" refers to an alkyl group as defined hereinafter that is substituted by a hydroxy group (-OH).

"Carbonyl" refers to the -C(=O)- group.

"Nitro" refers to -NO₂.

"Cyano" refers to -CN.

"Amino" refers to -NH₂.

"Substituted amino" refers to an amino group substituted by one or two alkyl, alkylcarbonyl, arylalkyl, or heteroarylalkyl groups as defined hereinafter, for example, monoalkylamino, dialkylamino, alkanoylamino, arylalkylamino, or heteroarylalkylamino.

"Carboxyl" refers to -COOH.

In the present application, as a group or part of another group (for example, as used in groups such as halo-substituted alkyl), the term "alkyl" refers to a fully saturated straight-chain or branched-chain hydrocarbon chain group consisting solely of carbon atoms and hydrogen atoms, having, for example, 1 to 12 (C₁-C₁₂ alkyl) (preferably 1 to 8 (C₁-C₈ alkyl), more preferably 1 to 6 (C₁-C₆ alkyl)) carbon atoms, and connected to the rest of the molecule by one or more single bonds, including, for example, but not limited to, methyl, ethyl, n-propyl, isopropyl, n-butyl, isobutyl, sec-butyl, tert-butyl, n-pentyl, 2-methylbutyl, 2,2-dimethylpropyl, n-hexyl, heptyl, 2-methylhexyl, 3-methylhexyl, octyl, nonyl, decyl, and the like. For the purposes of the present invention, the term "alkyl" preferably refers to an alkyl group containing ₁ to 6 carbon atoms.

In the present application, as a group or part of another group, the term "alkenyl" denotes a straight-chain or branched-chain hydrocarbon chain group consisting solely of carbon atoms and hydrogen atoms, containing at least one double bond, having, for example, 2 to 14 (C₂-C₁₄ alkenyl) (preferably 2 to 10 (C₂-C₁₀ alkenyl), more preferably 2 to 6 (C₂-C₆ alkenyl)) carbon atoms, and connected to the rest of the molecule by one or more single bonds, such as, but not limited to, vinyl, propenyl, allyl, but-1-enyl, but-2-enyl, pent-1-enyl, penta-1,4-dienyl, and the like.

As used herein, as a group or part of another group, the term "alkynyl" denotes a straight-chain or branched-chain hydrocarbon chain group consisting solely of carbon atoms and hydrogen atoms, containing at least one carbon-carbon triple bond, having, for example, 2 to 14 (C₂-C₁₄ alkynyl) (preferably 2 to 10 (C₂-C₁₀ alkynyl), more preferably 2 to 6 (C₂-C₆ alkynyl)) carbon atoms, and connected to the rest of the molecule by one or more single bonds, such as, but not limited to, ethynyl, 1-propynyl, 1-butynyl, heptynyl, octynyl, and the like.

In the present application, as a group or part of another group, the term "carbocycle (carbocyclyl)" denotes a stable, non-aromatic, monocyclic or polycyclic hydrocarbon group consisting solely of carbon atoms and hydrogen atoms, which may include fused ring systems, bridged ring systems, or spiro ring systems, having 3 to 15 carbon atoms (i.e., C₃-C₁₅ or 3- to 15-membered carbocycle (carbocyclyl)), preferably 3 to 10 carbon atoms (i.e., C₃-C₁₀ or 3- to 10-membered carbocycle (carbocyclyl)), more preferably 3 to 8 carbon atoms (i.e., C₃-C₈ or 3-to 8-membered carbocycle (carbocyclyl)), still more preferably 3 to 6 carbon atoms (i.e., C₃-C₆ or 3- to 6-membered carbocycle (carbocyclyl)), and which is a saturated or unsaturated ring (e.g., cycloalkyl, cycloalkenyl, etc.), and which may be connected to the rest of the molecule via any suitable carbon atom through one or more single bonds. Unless otherwise specifically indicated in this specification, carbon atoms in a carbocyclyl group may be optionally oxidized. Examples of carbocyclyl groups include, but are not limited to, cyclopropyl, cyclobutyl, cyclopentyl, cyclohexyl, cycloheptyl, cyclooctyl, adamantyl, 2,3-dihydro-1H-indenyl, octahydro-4,7-methano-1H-indenyl, 1,2,3,4-tetrahydronaphthyl, 5,6,7,8-tetrahydronaphthyl, cyclopentenyl, cyclohexenyl, cyclohexadienyl, 1H-indenyl, 8,9-dihydro-7H-benzocyclohepten-6-yl, 6,7,8,9-tetrahydro-5H-benzocycloheptenyl, 5,6,7,8,9,10-hexahydrobenzocyclooctenyl, fluorenyl, bicyclo[1.1.1]pentane, bicyclo[2.2.1]heptyl, 7,7-dimethylbicyclo[2.2.1]heptyl, bicyclo[2.2.1]heptenyl, bicyclo[2.2.2]octyl, bicyclo[3.1.1]heptyl, bicyclo[3.2.1]octyl, bicyclo[2.2.2]octenyl, bicyclo[3.2.1]octenyl, octahydro-2,5-methanopentalenyl, and the like.

In the present application, as a group or part of another group, the term "cycloalkyl" (e.g., "C₃-C₆ cycloalkyl") refers to the above-mentioned fully saturated carbocycle (carbocyclyl); typical cycloalkyl groups include, but are not limited to, cyclopropyl, cyclobutyl, cyclopentyl, cyclohexyl, cycloheptyl, cyclooctyl, adamantyl, and the like.

In the present application, as a group or part of another group, the term "cycloalkenyl" (e.g., "C₄-C₆ cycloalkenyl") refers to a partially unsaturated carbocycle (carbocyclyl); typical cycloalkenyl groups include, but are not limited to, cyclobutenyl, cyclopentenyl, cyclohexenyl, and the like.

In the present application, as a group or part of another group, the term "heterocycle (heterocyclyl)" denotes a stable 3- to 20-membered non-aromatic cyclic group consisting of 2 to 14 carbon atoms and 1 to 6 heteroatoms selected from nitrogen, phosphorus, oxygen, and sulfur (i.e., 3- to 20-membered heterocycle (heterocyclyl)). Unless otherwise specifically indicated in this specification, the heterocyclyl may be a monocyclic, bicyclic, tricyclic, or higher polycyclic ring system, which may include fused ring systems, bridged ring systems, or spiro ring systems; the nitrogen, carbon, or sulfur atom in the heterocyclyl may be optionally oxidized; the nitrogen atom may be optionally quaternized; and the heterocyclyl may be partially or fully saturated. The heterocyclyl may be connected to the rest of the molecule via a carbon atom or a heteroatom through one or more single bonds. In a heterocyclyl group containing fused rings, one or more rings may be an aryl or heteroaryl as defined hereinafter, provided that the point of attachment to the rest of the molecule is a non-aromatic ring atom. For the purposes of the present invention, heterocyclyl is preferably a stable 4- to 11-membered non-aromatic monocyclic, bicyclic, bridged, or spiro group containing 1 to 3 heteroatoms selected from nitrogen, oxygen, and sulfur (i.e., 4- to 11-membered heterocycle (heterocyclyl)), more preferably a stable 4- to 8-membered non-aromatic monocyclic, bicyclic, bridged, or spiro group containing 1 to 3 heteroatoms selected from nitrogen, oxygen, and sulfur (i.e., 4- to 8-membered heterocycle (heterocyclyl)). Examples of heterocyclyl groups include, but are not limited to: pyrrolidinyl, morpholinyl, piperazinyl, homopiperazinyl, piperidinyl, thiomorpholinyl, 2-azabicyclo[2.2.2]octanyl, 2,7-diazaspiro[3.5]nonan-7-yl, 2-oxa-6-azaspiro[3.3]heptan-6-yl, 2,5-diazabicyclo[2.2.1]heptan-2-yl, azetidinyl, pyranyl, tetrahydropyranyl, thiopyranyl, tetrahydrofuranyl, oxazinyl, dioxolanyl, tetrahydroisoquinolinyl, decahydroisoquinolinyl, imidazolinyl, imidazolidinyl, quinolizinyl, thiazolidinyl, isothiazolidinyl, isoxazolidinyl, dihydroindolyl, octahydroindolyl, octahydroisoindolyl, pyrrolidinyl, pyrazolidinyl, phthalimidyl, and the like.

In the present application, as a group or part of another group, the term "aryl" denotes a conjugated hydrocarbon ring system group having 6 to 18 carbon atoms (preferably 6 to 10 carbon atoms, i.e., C₆-C₁₀ aryl or 6- to 10-membered aryl). For the purposes of the present invention, aryl may be a monocyclic, bicyclic, tricyclic, or higher polycyclic ring system, and may also be fused to a carbocyclyl or heterocyclyl as defined above, provided that the aryl is connected to the rest of the molecule via an atom on the aromatic ring through one or more single bonds. Examples of aryl groups include, but are not limited to, phenyl, naphthyl, anthracenyl, phenanthrenyl, fluorenyl, 2,3-dihydro-1H-isoindolyl, 2-benzoxazolinone, 2H-1,4-benzoxazin-3(4H)-on-7-yl, and the like.

In the present application, the term "arylalkyl" refers to an alkyl group as defined above substituted by an aryl group as defined above.

In the present application, as a group or part of another group, the term "heteroaryl" denotes a 5- to 16-membered conjugated ring system group having 1 to 15 carbon atoms (preferably 1 to 10 carbon atoms) and 1 to 6 heteroatoms selected from nitrogen, oxygen, and sulfur in the ring. Unless otherwise specifically indicated in this specification, heteroaryl may be a monocyclic, bicyclic, tricyclic, or higher polycyclic ring system, and may also be fused to a carbocyclyl or heterocyclyl as defined above, provided that the heteroaryl is connected to the rest of the molecule via an atom on the heteroaromatic ring through one or more single bonds. The nitrogen, carbon, or sulfur atom in the heteroaryl may be optionally oxidized; the nitrogen atom may be optionally quaternized. For the purposes of the present invention, heteroaryl is preferably a stable 5- to 12-membered aromatic group containing 1 to 5 heteroatoms selected from nitrogen, oxygen, and sulfur, more preferably a stable 5- to 10-membered aromatic group containing 1 to 4 heteroatoms selected from nitrogen, oxygen, and sulfur (also referred to as 5 - to 10-membered heteroaryl), or a 5- to 6-membered aromatic group containing 1 to 3 heteroatoms selected from nitrogen, oxygen, and sulfur (also referred to as 5- to 6-membered heteroaryl or 5- or 6-membered heteroaryl). Examples of heteroaryl groups include, but are not limited to, thienyl, imidazolyl, pyrazolyl, thiazolyl, oxazolyl, oxadiazolyl, isoxazolyl, pyridyl, pyrimidinyl, pyrazinyl, pyridazinyl, benzimidazolyl, benzopyrazolyl, indolyl, furanyl, pyrrolyl, triazolyl, tetrazolyl, triazinyl, indolizinyl, isoindolyl, indazolyl, isoindazolyl, purinyl, quinolinyl, isoquinolinyl, phthalazinyl, naphthyridinyl, quinoxalinyl, pteridinyl, carbazolyl, carbolinyl, phenanthridinyl, phenanthrolinyl, acridinyl, phenazinyl, isothiazolyl, benzothiazolyl, benzothienyl, oxatriazolyl, cinnolinyl, quinazolinyl, phenylthio, indolizinyl, 1,10-phenanthrolinyl, isoxazolyl, phenoxazinyl, phenothiazinyl, 4,5,6,7-tetrahydrobenzo[b]thienyl, naphthopyridinyl, [1,2,4]triazolo[4,3-b]pyridazine, [1,2,4]triazolo[4,3-a]pyrazine, [1,2,4]triazolo[4,3-c]pyrimidine, [1,2,4]triazolo[4,3-a]pyridine, imidazo[1,2-a]pyridine, imidazo[1,2-b]pyridazine, imidazo[1,2-a]pyrazine, and the like.

In the present application, the term "heteroarylalkyl" refers to an alkyl group as defined above substituted by a heteroaryl group as defined above.

In the present application, the term "absent" means that the two sides of the group defined above are directly connected by a chemical bond. For example, "in A-B-C, B is absent" represents "A-C".

In the present application, " 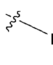 R" of " 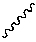 " represents the attachment position of the group R.
In the present application, except as specifically indicated in the claims, the terms "optionally" and "optional" mean that the subsequently described event or condition may or may not occur, and that said description encompasses both the occurrence and non-occurrence of said event or condition. For example, "optionally substituted aryl" means that the hydrogens on the aryl may be substituted or unsubstituted, and said description encompasses both substituted aryl and unsubstituted aryl. For example, in the absence of an explicit listing of substituents, the terms "optionally substituted", "substituted", or "substituted by..." as used herein mean that one or more hydrogen atoms on a given atom or group are independently substituted by one or more, for example, 1, 2, 3, or 4 substituents, said substituents being independently selected from: deuterium (D), halo, -OH, oxo (=O), mercapto, cyano, -CD₃, -C₁-C₆ alkyl (preferably -C₁-C₃ alkyl), C₂-C₆ alkenyl, C₂-C₆ alkynyl, cycloalkyl (preferably C₃-C₈ cycloalkyl), aryl, heterocyclyl (preferably 3- to 8-membered heterocyclyl), heteroaryl, aryl-C₁-C₆ alkyl-, heteroaryl-C₁-C₆ alkyl-, C₁-C₆ haloalkyl-, -OC₁-C₆ alkyl (preferably -OC₁-C₃ alkyl), -OC₂-C₆ alkenyl, -O cycloalkyl, -O heterocyclyl, -O aryl, -O heteroaryl, -OC₁-C₆ alkyl-phenyl, -C₁-C₆ alkyl-OH (preferably -C₁-C₄ alkyl-OH), -C₁-C₆ alkyl-SH, -C₁-C₆ alkyl-O-C₁-C₆ alkyl, -OC₁-C₆ haloalkyl, -NH₂, -C₁-C₆ alkyl-NH₂ (preferably -C₁-C₃ alkyl-NH₂), -N(C₁-C₆ alkyl)₂ (preferably -N(C₁-C₃ alkyl)₂), -NH(C₁-C₆ alkyl) (preferably -NH(C₁-C₃ alkyl)), -N(C₁-C₆ alkyl)(C₁-C₆ alkyl-phenyl), -NH(C₁-C₆ alkyl-phenyl), -N(C₁-C₆ alkyl)(aryl), -NH(aryl), nitro, -C(O)-OH, -C(O)OC₁-C₆ alkyl (preferably -C(O)OC₁-C₃ alkyl), -CONRⁱRⁱⁱ (wherein Rⁱ and Rⁱⁱ are H, D, and C₁-C₆ alkyl, preferably C₁-C₃ alkyl), -NHC(O)(C₁-C₆ alkyl), -NHC(O)(phenyl), -N(C₁-C₆ alkyl)C(O)(C₁-C₆ alkyl), -N(C₁-C₆ alkyl)C(O)(phenyl), -C(O)C₁-C₆ alkyl, -C(O)heteroaryl (preferably -C(O)-5- to 7-membered heteroaryl), -C(O)C₁-C₆ alkyl-phenyl, -C(O)C₁-C₆ haloalkyl, -OC(O)C₁-C₆ alkyl (preferably -OC(O)C₁-C₃ alkyl), -S(O)₂-C₁-C₆ alkyl, -S(O)-C₁-C₆ alkyl, -S(O)₂-phenyl, -S(O)₂-C₁-C₆ haloalkyl, -S(O)₂NH₂, -S(O)₂NH(C₁-C₆ alkyl), -S(O)₂NH(phenyl), -NHS(O)₂(C₁-C₆ alkyl), -NHS(O)₂(phenyl), and -NHS(O)₂(C₁-C₆ haloalkyl), wherein each of said alkyl, alkenyl, alkynyl, cycloalkyl, phenyl, aryl, heterocyclyl, and heteroaryl is optionally further substituted by one or more substituents selected from: halo, -OH, oxo (=O), -NH₂, cycloalkyl, 3- to 8-membered heterocyclyl, C₁-C₄ alkyl, C₁-C₄ haloalkyl-, -OC₁-C₄ alkyl, -C₁-C₄ alkyl-OH, -C₁-C₄ alkyl-O-C₁-C₄ alkyl, -OC₁-C₄ haloalkyl, cyano, nitro, -C(O)-OH, -C(O)OC₁-C₆ alkyl, -CON(C₁-C₆ alkyl)₂, -CONH(C₁-C₆ alkyl), -CONH₂, -NHC(O)(C₁-C₆ alkyl), -NH(C₁-C₆ alkyl)C(O)(C₁-C₆ alkyl), -SO₂(C₁-C₆ alkyl), -SO₂(phenyl), -SO₂(C₁-C₆ haloalkyl), -SO₂NH₂, -SO₂NH(C₁-C₆ alkyl), -SO₂NH(phenyl), -NHSO₂(C₁-C₆ alkyl), -NHSO₂(phenyl), and -NHSO₂(C₁-C₆ haloalkyl). When an atom or group is substituted by multiple substituents, said substituents may be the same or different. The terms "moiety", "structural moiety", "chemical moiety", "group", and "chemical group" as used herein refer to a specific fragment or functional group in a molecule. Chemical moieties are generally regarded as chemical entities embedded in or appended to a molecule.

**In** the present invention, (C₁-C₄ alkyl)₂ amino represents an amine substituted by two C₁-C₄ alkyl groups, which may be, for example, 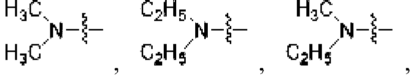 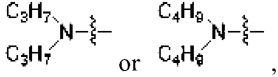 and the like.

In the present invention, "multiple" refers to 2, 3, or 4.

### Active Ingredient

As used herein, the term "compound of the present invention" or "active ingredient" refers to the compound of Formula I, and also encompasses a pharmaceutically acceptable salt, enantiomer, diastereomer, tautomer, cis-trans isomer, solvate, polymorph, deuterated form thereof, or a combination thereof.

"Stereoisomers" refers to compounds composed of the same atoms, bonded by the same bonds, but having different three-dimensional structures. The present invention shall encompass various stereoisomers and mixtures thereof.

When a compound of the present invention contains an olefinic double bond, unless otherwise specified, the compounds of the present invention are intended to encompass both E- and Z-geometric isomers.

"Tautomers" refers to isomers formed by the transfer of a proton from one atom of a molecule to another atom of the same molecule. All tautomeric forms of the compounds of the present invention shall also be encompassed within the scope of the present invention.

The compounds of the present invention, or pharmaceutically acceptable salts thereof, may contain one or more chiral carbon atoms and may therefore give rise to enantiomers, diastereomers, and other stereoisomeric forms. Each chiral carbon atom may be defined as (R)- or (S)- based on stereochemistry. The present invention is intended to include all possible isomers, as well as racemates and optically pure forms thereof. The preparation of the compounds of the present invention may employ racemates, diastereomers, or enantiomers as starting materials or intermediates. Optically active isomers may be prepared using chiral synthons or chiral reagents, or resolved using conventional techniques, such as crystallization and chiral chromatography.

Conventional techniques for the preparation/separation of individual isomers include chiral synthesis from suitable optically pure precursors, or resolution of the racemate (or of a racemate of a salt or derivative) using, for example, chiral high-performance liquid chromatography; see, e.g., Gerald Gübitz and Martin G. Schmid (eds.), Chiral Separations, Methods and Protocols, Methods in Molecular Biology, Vol. 243, 2004; A.M. Stalcup, Chiral Separations, Annu. Rev. Anal. Chem. 3:341-63, 2010; Fumiss et al. (eds.), VOGEL'S ENCYCLOPEDIA OF PRACTICAL ORGANIC CHEMISTRY, 5TH ED., Longman Scientific and Technical Ltd., Essex, 1991, 809-816; Heller, Acc. Chem. Res. 1990, 23, 128.

In the present application, the term "pharmaceutically acceptable salt" includes pharmaceutically acceptable acid addition salts and pharmaceutically acceptable base addition salts.

"Pharmaceutically acceptable acid addition salt" refers to a salt formed with an inorganic or organic acid that is capable of retaining the biological effectiveness of the free base without producing other adverse effects. Inorganic acid salts include, but are not limited to, hydrochloride, hydrobromide, sulfate, nitrate, phosphate, and the like; organic acid salts include, but are not limited to, formate, acetate, 2,2-dichloroacetate, trifluoroacetate, propionate, hexanoate, octanoate, decanoate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, mesylate, besylate, p-toluenesulfonate, alginate, ascorbate, salicylate, 4-aminosalicylate, naphthalenedisulfonate, and the like. These salts may be prepared by methods known in the art.

"Pharmaceutically acceptable base addition salt" refers to a salt formed with an inorganic or organic base that is capable of retaining the biological effectiveness of the free acid without producing other adverse effects. Salts derived from inorganic bases include, but are not limited to, sodium, potassium, lithium, ammonium, calcium, magnesium, iron, zinc, copper, manganese, and aluminum salts, and the like. Preferred inorganic salts are the ammonium, sodium, potassium, calcium, and magnesium salts. Salts derived from organic bases include, but are not limited to, salts of: primary, secondary, and tertiary amines, substituted amines including naturally occurring substituted amines, cyclic amines, and basic ion-exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, and the like. Preferred organic bases include isopropylamine, diethylamine, ethanolamine, trimethylamine, dicyclohexylamine, choline, and caffeine. These salts may be prepared by methods known in the art.

### Pharmaceutical Compositions and Methods of Administration

Because the compounds of the present invention exhibit excellent inhibitory activity against methionine adenosyltransferase 2A (MAT2A), the compounds of the present invention, the various crystal forms, pharmaceutically acceptable inorganic or organic salts, hydrates, or solvates thereof, as well as pharmaceutical compositions containing a compound of the present invention as the principal active ingredient, can be used for the treatment, prevention, and amelioration of MTAP-deleted (MTAP^{-/-}) cancers or tumors (for example, the specific tumors listed elsewhere herein).

The pharmaceutical compositions of the present invention comprise a compound of the present invention or a pharmacologically acceptable salt thereof in a safe and effective amount, together with a pharmacologically acceptable excipient or carrier. As used herein, "safe and effective amount" means an amount of the compound that is sufficient to provide a marked improvement in the condition without producing serious side effects. Generally, the pharmaceutical composition contains 1-2000 mg of the compound of the present invention per dose, more preferably 10-500 mg of the compound of the present invention per dose. Preferably, the "dose" is one capsule or tablet.

In the present application, "pharmaceutical composition" refers to a formulation of a compound of the present invention with a medium generally accepted in the art for delivering a biologically active compound to a mammal (e.g., a human). Said medium includes a pharmaceutically acceptable carrier. The purpose of a pharmaceutical composition is to facilitate administration to an organism and to promote absorption of the active ingredient so as to exert its biological activity

The compounds of general formula (I) may be used in combination with other drugs that are known to treat or ameliorate similar conditions. When co-administered, the route of administration and dosage of the original drug may remain unchanged, while the compound of Formula I is administered concurrently or subsequently. When a compound of Formula I is administered concurrently with one or more other drugs, a pharmaceutical composition comprising both the compound of Formula I and one or more known drugs may preferably be used. Drug combinations also include the administration of a compound of Formula I and one or more other known drugs over overlapping time periods. When a compound of Formula I is used in combination with one or more other drugs, the dose of the compound of Formula I or of the known drug may be lower than when each is used alone.

Drugs or active ingredients that may be used in combination with a compound of general formula (I) include, but are not limited to: chemotherapeutic agents (e.g., docetaxel, paclitaxel, gemcitabine, and the like), PD-1 inhibitors (e.g., nivolumab, pembrolizumab, and the like), PD-L1 inhibitors (e.g., durvalumab, atezolizumab, and the like), CD47 antibodies (e.g., Hu5F9-G4, CC-90002, and the like), CD20 antibodies (e.g., rituximab, ibritumomab tiuxetan, and the like), KRAS inhibitors (e.g., AMG510 and the like), ALK inhibitors (e.g., ceritinib, alectinib, brigatinib, lorlatinib, ensartinib), EGFR inhibitors (e.g., afatinib, gefitinib, erlotinib, lapatinib, dacomitinib, icotinib, osimertinib, and the like), VEGFR inhibitors (e.g., sorafenib, pazopanib, regorafenib, cabozantinib, sunitinib, and the like), PI3K inhibitors (e.g., Dactolisib, Taselisib, and the like), BTK inhibitors (e.g., ibrutinib, tirabrutinib, acalabrutinib, zanubrutinib, vecabrutinib, and the like), HDAC inhibitors (e.g., Vorinostat, Fimepinostat, Givinostat, Tucidinostat, and the like), CDK inhibitors (e.g., palbociclib, Ribociclib, Abemaciclib, and the like), MEK inhibitors (e.g., selumetinib (AZD6244), Trametinib, and the like), ERK inhibitors (e.g., BVD523, HH2710, and the like), mTOR inhibitors (e.g., Vistusertib and the like), SHP2 inhibitors (e.g., RMC-4630, JAB-3068), SOS1 inhibitors (e.g., BI1701963 and the like), PRMT inhibitors, or combinations thereof.

As used herein, the term "pharmaceutically acceptable" refers to a substance (e.g., a carrier or diluent) that does not affect the biological activity or properties of the compound of the present invention and that is relatively non-toxic, i.e., the substance may be administered to a subject without causing an adverse biological response or interacting in an adverse manner with any of the components contained in the composition.

In the present application, "pharmaceutically acceptable carrier" includes, but is not limited to, any adjuvant, carrier, excipient, glidant, sweetener, diluent, preservative, dye/colorant, flavoring agent, surfactant, wetting agent, dispersing agent, suspending agent, stabilizer, isotonic agent, solvent, or emulsifier that is approved by the relevant governmental regulatory authorities as being acceptable for use in humans or domestic animals.

The mode of administration of the compounds or pharmaceutical compositions of the present invention is not particularly limited; representative modes of administration include, but are not limited to: oral, intratumoral, rectal, parenteral (intravenous, intramuscular, or subcutaneous), and topical administration.

Solid dosage forms for oral administration include capsules, tablets, pills, powders, and granules. In such solid dosage forms, the active compound is admixed with at least one conventional inert excipient (or carrier), such as sodium citrate or dicalcium phosphate, or with the following components: (a) fillers or extenders, for example, starch, lactose, sucrose, glucose, mannitol, and silicic acid; (b) binders, for example, hydroxymethylcellulose, alginates, gelatin, polyvinylpyrrolidone, sucrose, and acacia; (c) humectants, for example, glycerol; (d) disintegrants, for example, agar, calcium carbonate, potato or tapioca starch, alginic acid, certain complex silicates, and sodium carbonate; (e) solution retarders, for example, paraffin; (f) absorption accelerators, for example, quaternary ammonium compounds; (g) wetting agents, for example, cetyl alcohol and glyceryl monostearate; (h) adsorbents, for example, kaolin; and (i) lubricants, for example, talc, calcium stearate, magnesium stearate, solid polyethylene glycols, sodium lauryl sulfate, or mixtures thereof. In capsules, tablets, and pills, the dosage forms may also contain buffering agents.

Solid dosage forms such as tablets, dragees, capsules, pills, and granules may be prepared with coatings and shells, such as enteric coatings and other materials well known in the art. They may contain opacifying agents, and the release of the active compound or compounds in such compositions may be in a delayed manner in a certain portion of the digestive tract. Examples of embedding components that may be employed are polymeric substances and waxes. If necessary, the active compounds may also be formulated in microencapsulated form with one or more of the above-mentioned excipients.

Liquid dosage forms for oral administration include pharmaceutically acceptable emulsions, solutions, suspensions, syrups, or tinctures. Besides the active compound, the liquid dosage forms may contain inert diluents conventionally employed in the art, such as water or other solvents, solubilizers, and emulsifiers, for example, ethanol, isopropanol, ethyl carbonate, ethyl acetate, propylene glycol, 1,3-butylene glycol, dimethylformamide, as well as oils, particularly cottonseed oil, peanut oil, corn germ oil, olive oil, castor oil, sesame oil, or mixtures of these substances, and the like.

In addition to these inert diluents, the compositions may also contain adjuvants such as wetting agents, emulsifying and suspending agents, sweetening agents, flavoring agents, and perfuming agents.

In addition to the active compound, the suspensions may contain suspending agents, for example, ethoxylated isostearyl alcohols, polyoxyethylene sorbitol and sorbitan esters, microcrystalline cellulose, aluminum methoxide, and agar, or mixtures of these substances, and the like.

Compositions for parenteral injection may comprise physiologically acceptable sterile aqueous or non-aqueous solutions, dispersions, suspensions, or emulsions, and sterile powders for reconstitution into sterile injectable solutions or dispersions. Suitable aqueous and non-aqueous carriers, diluents, solvents, or excipients include water, ethanol, polyols, and suitable mixtures thereof.

The "tumor (cancer)" referred to in the present invention (e.g., an MTAP-deleted tumor (cancer) or an MTAP^{-/-} associated tumor (cancer)) includes, but is not limited to, diseases such as glioma, mesothelioma, esophageal cancer, bladder cancer, urothelial carcinoma, pancreatic cancer, melanoma, non-small cell lung cancer, head and neck cancer, sarcoma, cholangiocarcinoma, liver cancer, breast cancer, skin cancer, intestinal cancer, gastric cancer, renal cancer, urethral cancer, prostate cancer, testicular cancer, uterine cancer, ovarian cancer, vaginal cancer, fallopian tube cancer, multiple myeloma, spinal cord neurofibroma, astrocytoma, acute lymphoblastic leukemia, chronic lymphocytic leukemia, Hodgkin lymphoma, and non-Hodgkin lymphoma. As used herein, the terms "preventive", "prevention", and "prevent" include reducing in a subject the likelihood of the occurrence or worsening of a disease or condition.

As used herein, the term "treatment" and other similar synonymous terms include the following meanings:
(i) preventing the occurrence of a disease or condition in a mammal, particularly when such mammal is predisposed to said disease or condition but has not yet been diagnosed as having it;
(ii) inhibiting the disease or condition, i.e., arresting its development;
(iii) relieving the disease or condition, i.e., causing regression of said disease or condition; or
(iv) alleviating the symptoms caused by said disease or condition.

As used herein, the terms "effective amount", "therapeutically effective amount", or "pharmaceutically effective amount" refer to an amount of at least one agent or compound that, upon administration, is sufficient to alleviate, to some extent, one or more symptoms of the disease or condition being treated. The result may be a reduction and/or alleviation of signs, symptoms, or causes, or any other desired change in a biological system. For example, an "effective amount" for treatment is the amount of a composition comprising a compound disclosed herein that is required to provide clinically significant relief of the condition. Techniques such as dose-escalation trials may be used to determine an effective amount suitable for any individual case.

As used herein, the terms "administer", "administration", "administering", and the like refer to methods capable of delivering a compound or composition to a desired site of biological action. These methods include, but are not limited to, oral routes, transduodenal routes, parenteral injection (including intravenous, subcutaneous, intraperitoneal, intramuscular, or intra-arterial injection or infusion), topical administration, and rectal administration. Those skilled in the art are familiar with the administration techniques applicable to the compounds and methods described herein, for example, those discussed in Goodman and Gilman, The Pharmacological Basis of Therapeutics, current edition, Pergamon; and Remington's Pharmaceutical Sciences (current edition), Mack Publishing Co., Easton, Pa. In a preferred embodiment, the compounds and compositions discussed herein are administered orally.

As used herein, the terms "drug combination", "combination therapy", "combined administration", "administering additional therapy", "administering additional therapeutic agent", and the like refer to pharmaceutical therapy obtained by mixing or combining more than one active ingredient, including both fixed and non-fixed combinations of the active ingredients. The term "fixed combination" refers to the simultaneous administration to a patient of at least one compound described herein and at least one synergistic agent in the form of a single entity or single dosage form. The term "non-fixed combination" refers to the simultaneous, concurrent, or sequential administration at variable intervals, in the form of separate entities, of at least one compound described herein and at least one synergistic preparation. These also apply to cocktail therapy, e.g., the administration of three or more active ingredients.

The pharmaceutical compositions of the present invention comprise a compound of the present invention or a pharmacologically acceptable salt thereof in a safe and effective amount, together with a pharmacologically acceptable excipient or carrier. As used herein, "safe and effective amount" means an amount of the compound that is sufficient to provide a marked improvement in the condition without producing serious side effects. Generally, the pharmaceutical composition contains 1-2000 mg of the compound of the present invention per dose, more preferably 10-1000 mg of the compound of the present invention per dose. Preferably, the "dose" is one capsule or tablet.

Compared with the Prior Art, the Present Invention Has the Following Beneficial Effects:
(1) The present invention provides a compound of Formula I having a novel structure, or a pharmaceutically acceptable salt thereof;
(2) The compounds of the present invention are capable of inhibiting MAT2A, and can be used in the preparation of medicaments for treating MTAP^{-/-} associated cancers.
(3) The compounds of the present invention exhibit excellent proliferation-inhibitory activity against MTAP-deleted (MTAP^{-/-}) cells.
(4) The compounds of the present invention exhibit excellent selective cell-proliferation-inhibitory activity; the proliferation-inhibitory activity of the compounds of the present application against MTAP-deleted (MTAP^{-/-}) cells is significantly superior to that against MTAP-non-deleted (MTAP^{WT}) cells, and they exhibit essentially no proliferation-inhibitory activity against MTAP-non-deleted (MTAP^{WT}) cells.

The present invention is further illustrated below in conjunction with specific examples. It is to be understood that these examples are intended only to illustrate the present invention and are not intended to limit the scope of the present invention. Experimental methods in the following examples for which specific conditions are not specified are generally carried out under conventional conditions or under the conditions recommended by the manufacturer. Unless otherwise specified, percentages and parts are by weight.

The experimental materials and reagents used in the following examples, unless otherwise specified, are commercially available.

In the various examples, ¹H NMR spectra were recorded on a Bruker AVANCE NEO 400 MHz NMR spectrometer, with chemical shifts expressed in δ (ppm); liquid chromatography-mass spectrometry (LCMS) was recorded on a Shimadzu LC-20AD, SIL-20A, CTO-20AC, SPD-M20A, CBM-20A, LCMS-2020 mass spectrometer; preparative HPLC separations were performed using a Gilson-281 liquid chromatograph.

### Example 1: Synthesis of Compound 1

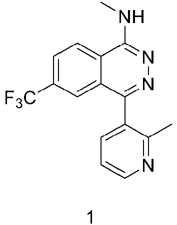

The synthetic route for Compound 1 is shown below: 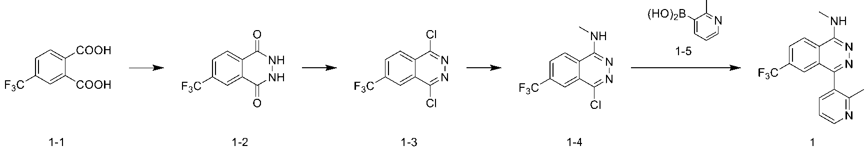
(1) Compound 1-1 (5 g, 21.36 mmol) was added to thionyl chloride (15 mL) and heated to reflux for 2 hours. The reaction mixture was concentrated under reduced pressure, and the residue was dissolved in acetic acid (30 mL); anhydrous hydrazine (2.89 mL, 92.90 mmol) was then added, and the mixture was heated to reflux at 120 °C overnight. The reaction mixture was concentrated to dryness directly, water (20 mL) was added, and a solid precipitated. The mixture was filtered, and the filter cake was filtered under reduced pressure and then dried in vacuo overnight to afford Compound 1-2. MS-ESI [M+H]⁺, calc. 231, found 231.
(2) Compound 1-2 (3 g, 13.03 mmol) was dissolved in phosphorus oxychloride (30 mL), and the mixture was stirred at 100 °C overnight. The reaction mixture was concentrated under reduced pressure to remove phosphorus oxychloride, and the resulting crude oil was slowly added dropwise to ice water (50 mL) with thorough stirring, adjusted to pH = 8 with 2 mol/L aqueous sodium hydroxide solution, and extracted with ethyl acetate (50 mL × 3). The combined organic layers were washed with saturated brine (50 mL × 3), dried over anhydrous sodium sulfate, and filtered, and the filtrate was concentrated under reduced pressure to remove the solvent. Purification by silica gel column chromatography (petroleum ether : ethyl acetate = 10:1) afforded Compound 1-3. MS-ESI [M+H]⁺, calc. 267, found 267.
(3) To a solution of Compound 1-3 (1500 mg, 5.62 mmol) in N,N-dimethylformamide (20 mL) were added methylamine hydrochloride (379.28 mg, 5.62 mmol) and sodium carbonate (1964.76 mg, 18.54 mmol). The mixture was stirred at 120 °C overnight. The reaction mixture was concentrated under reduced pressure, and the crude product was purified by silica gel column chromatography (petroleum ether : ethyl acetate = 1:4) to afford Compound 1 -4. MS-ESI [M+H]⁺, calc. 262, found 262.
(4) To a solution of Compound 1-4 (95 mg, 0.36 mmol) in 1,4-dioxane (4 mL) and water (1 mL) were added Compound 1-5 (61.16 mg, 0.45 mmol), sodium carbonate (76.97 mg, 0.73 mmol), and bis(diphenylphosphino)ferrocene-palladium(II) dichloride (26.94 mg, 0.04 mmol). The mixture was stirred at 110 °C overnight under nitrogen protection. The reaction mixture was concentrated under reduced pressure, and the crude product was purified by high-performance liquid chromatography (Puningtech-Pntulips-C18-5 µm-21.2 × 150 mm, A: 0.05% trifluoroacetic acid/water; B: acetonitrile, 0-32% B) to afford Compound 1. MS-ESI [M+H]⁺, calc. 319, found 319. ¹H NMR (400 MHz, DMSO-d6) δ 10.35 (s, 1H), 8.95-8.73 (m, 2H), 8.57 (d, J = 8.7 Hz, 1H), 8.01 (s, 1H), 7.76 (s, 1H), 7.67-7.46 (m, 1H), 3.24 (d, J = 3.5 Hz, 3H), 2.36 (d, J = 15.8 Hz, 3H).

### Example 2: Synthesis of Compound 2

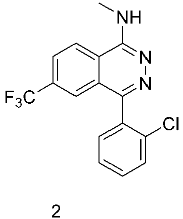

The synthetic route for Compound 2 is shown below:

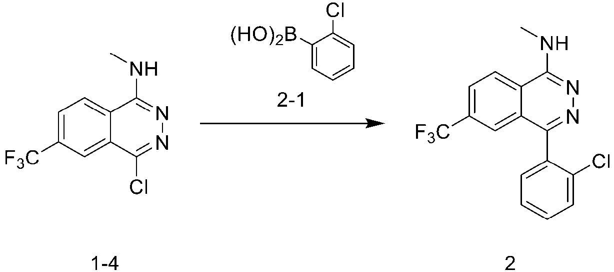

To a solution of Compound 1-4 (95 mg, 0.36 mmol) in 1,4-dioxane (4 mL) and water (1 mL) were added Compound 2-1 (56 mg, 0.36 mmol), sodium carbonate (76.97 mg, 0.73 mmol), and bis(diphenylphosphino)ferrocene-palladium(II) dichloride (26.94 mg, 0.04 mmol). The mixture was stirred at 110 °C overnight under nitrogen protection. The reaction mixture was concentrated under reduced pressure, and the crude product was purified by high-performance liquid chromatography (Puningtech-Pntulips C18 5 µm-21.2 × 150 mm, A: 0.1% formic acid/water; B: acetonitrile, 0-47% B) to afford Compound 2. MS-ESI [M+H]⁺, calc. 338, found 338. ¹H NMR (400 MHz, DMSO-d6) δ 8.57 (d, J = 8.7 Hz, 1H), 8.27 (d, J = 7.6 Hz, 1H), 8.15-8.01 (m, 1H), 7.69 (d, J = 7.6 Hz, 1H), 7.64-7.53 (m, 3H), 7.49 (s, 1H), 3.12 (d, J = 4.3 Hz, 3H).

### Example 3: Synthesis of Compound 3

Synthesized by reference to Example 1, replacing methylamine with deuterated methylamine, to afford Compound 3.

| | | |
|---|---|---|
| 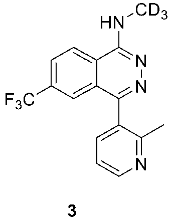 | MS-ESI [M+H]⁺, calc. 322, found 322. | ¹H NMR (400 MHz, DMSO-d6) δ 8.85 (s, 1H), 8.63 (dd, J = 4.8, 1.8 Hz, 1H), 8.15 - 8.00 (m, 2H), 7.75 (dd, J = 7.6, 1.8 Hz, 1H), 7.52 (d, J = 8.6 Hz, 1H), 7.42 (dd, J = 7.6, 5.0 Hz, 1H), 2.23 (s, 3H). |

### Example 4: Synthesis of Compound 4

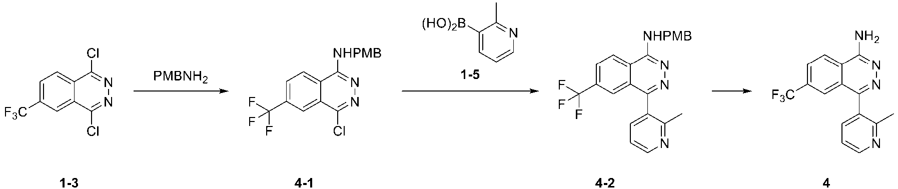

(1) To a solution of Compound 1-3 (500 mg, 1.87 mmol) in dimethyl sulfoxide (7 mL) were added 4-methoxybenzylamine (256.86 mg, 1.87 mmol) and sodium carbonate (496.15 mg, 4.68 mmol). The mixture was stirred at 130 °C overnight. The reaction mixture was poured into water and extracted with ethyl acetate; the organic phase was washed with saturated brine, dried, and concentrated. The crude product was purified by silica gel column chromatography (ethyl acetate : petroleum ether = 6%-10%) to afford Compound 4-1. MS-ESI [M+H]⁺, calc. 368, found 368.
(2) To a solution of Compound 4-1 (607 mg, 1.65 mmol) in 1,4-dioxane (10 mL) and water (2.2 mL) were added Compound 1-5 (294.32 mg, 2.15 mmol), sodium carbonate (438.07 mg, 4.13 mmol), and bis(diphenylphosphino)ferrocene-palladium(II) dichloride (245.27 mg, 0.33 mmol). The mixture was stirred at 110 °C overnight under nitrogen protection. The reaction mixture was concentrated under reduced pressure, and the crude product was purified by silica gel column chromatography (methanol/dichloromethane = 2%-3%) to afford Compound 4-2. MS-ESI [M+H]⁺, calc. 425, found 425.
(3) At 0 °C, Compound 4-2 (400 mg, 0.94 mmol) was dissolved in a mixed solution of trifluoroacetic acid (4 mL) and trifluoromethanesulfonic acid (0.4 mL). The mixture was stirred for an additional 10 minutes under nitrogen protection, and the reaction was monitored to completion by LCMS. The reaction mixture was concentrated, and the crude product was purified by preparative reverse-phase column chromatography (Waters-CORTECS C18 2.7 µm-4.6 × 30 mm; 25 mL/min; 25 °C; 0%-100% acetonitrile/0.05% NH4HCO3 aqueous buffer) to afford Compound 4. MS-ESI [M+H]⁺, calc. 305, found 305. ¹H NMR (400 MHz, DMSO-d6) δ 8.70-8.52 (m, 2H), 8.25 (dd, J = 8.8, 1.8 Hz, 1H), 7.78 (dd, J = 7.6, 1.8 Hz, 1H), 7.59-7.35 (m, 4H), 2.23 (s, 3H).

### Example 5: Synthesis of Compound 5

Synthesized by reference to Example 4, replacing 1-5 with the corresponding pyridineboronic acid, to afford Compound 5.

| | | |
|---|---|---|
| 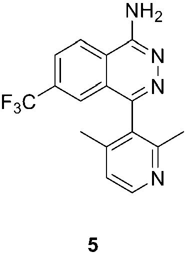 | MS-ESI [M+H]⁺, calc. 319, found 319. | ¹H NMR (400 MHz, CDCl3) δ 8.55 (d, J = 5.2 Hz, 1H), 8.13 - 8.03 (m, 2H), 7.59 (d, J = 0.8 Hz, 1H), 7.19 (d, J = 5.2 Hz, 1H), 5.88 (s, 2H), 2.22 (s, 3H), 2.02 (s, 3H). |

### Example 6: Synthesis of Compound 6

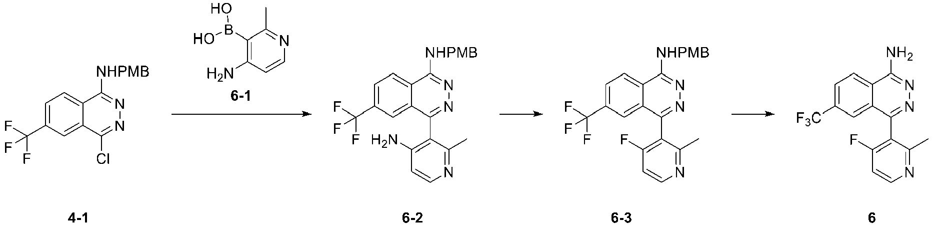

(1) To a solution of Compound 4-1 (200 mg, 0.54 mmol) in 1,4-dioxane (10 mL) and water (2.2 mL) were added Compound 6-1 (206.60 mg, 0.82 mmol), sodium carbonate (115.28 mg, 1.09 mmol), and methanesulfonato(2-dicyclohexylphosphino-2',4',6'-tri-isopropyl-1,1'-biphenyl)(2'-amino-1,1' -biphenyl-2-yl)palladium(II) (92.50 mg, 0.11 mmol). The mixture was stirred at 110 °C overnight under nitrogen protection. The reaction mixture was concentrated under reduced pressure, and the crude product was purified by silica gel column chromatography (methanol/dichloromethane = 2%-3%) to afford Compound 6-2. MS-ESI [M+H]⁺, calc. 440, found 440.
(2) At -10 °C, a solution of sodium nitrite (29.91 mg, 0.43 mmol) in water (1 mL) was added dropwise to a solution of Compound 6-2 (127 mg, 0.29 mmol) in HF·pyridine (3 mL). After the addition was complete, the reaction was stirred at 20 °C for 1 hour. The reaction was monitored by LCMS; the reaction mixture was poured into saturated aqueous sodium bicarbonate solution and extracted with ethyl acetate. The organic phase was washed with saturated brine, dried, and concentrated. The crude product was purified by silica gel column chromatography with a gradient (methanol/dichloromethane = 2%-3%) to afford Compound 6-3. MS-ESI [M+H]⁺, calc. 443, found 443.
(3) At 0 °C, Compound 6-3 (95 mg, 0.21 mmol) was dissolved in a mixed solution of trifluoroacetic acid (4 mL) and trifluoromethanesulfonic acid (0.4 mL). The mixture was stirred for an additional 10 minutes under nitrogen protection, and the reaction was monitored to completion by LCMS. The reaction mixture was concentrated, and the crude product was purified by preparative reverse-phase column chromatography (Waters-CORTECS C18 2.7 µm-4.6 × 30 mm; 25 mL/min; 25 °C; 0%-100% acetonitrile/0.05% NH4HCO3 aqueous buffer) to afford Compound 6. MS-ESI [M+H]⁺, calc. 323, found 323. ¹H NMR (400 MHz, CDCl3) δ 8.68 (dd, J = 8.4, 5.6 Hz, 1H), 8.18-8.04 (m, 2H), 7.68 (s, 1H), 7.11 (dd, J = 8.4, 5.6 Hz, 1H), 5.96 (s, 2H), 2.40 (s, 3H).

### Example 7: Synthesis of Compound 7

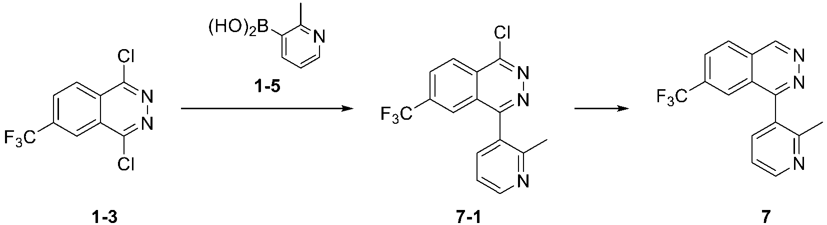

(1) To a solution of Compound 1-3 (550 mg, 2.06 mmol) in 1,4-dioxane (10 mL) and water (2.2 mL) were added Compound 1-5 (276.41 mg, 2.02 mmol), sodium carbonate (545.77 mg, 5.15 mmol), and bis(diphenylphosphino)ferrocene-palladium(II) dichloride (305.57 mg, 0.41 mmol). The mixture was stirred at 110 °C for 3 hours under nitrogen protection. The reaction mixture was concentrated under reduced pressure, and the crude product was purified by silica gel column chromatography (methanol/dichloromethane = 2%-3%) to afford Compound 7-1. MS-ESI [M+H]⁺, calc. 324, found 324.
(2) Under nitrogen protection, Compound 7-1 (249 mg, 0.77 mmol), tetrakis(triphenylphosphine)palladium (177.78 mg, 0.15 mmol), triethylamine (1.28 mL, 9.23 mmol), and formic acid (0.04 mL, 0.92 mmol) were dissolved in N,N-dimethylformamide (3 mL). The mixture was stirred at 110 °C for 40 minutes under nitrogen protection, and the reaction was monitored to completion by LCMS. The crude product was purified by preparative reverse-phase column chromatography (Waters-CORTECS-C18-2.7 µm-4.6 × 30 mm; 25 mL/min; 25 °C; 0%-100% acetonitrile/0.05% NH4HCO3 aqueous buffer) to afford Compound 7. MS-ESI [M+H]⁺, calc. 290, found 290. ¹H NMR (400 MHz, CDCl3) δ 9.64 (d, J = 0.8 Hz, 1H), 8.70 (dd, J = 4.8, 1.6 Hz, 1H), 8.17 (d, J = 8.4 Hz, 1H), 8.09 (dd, J = 8.4, 1.6 Hz, 1H), 7.83-7.80 (m, 1H), 7.66 (dd, J = 7.6, 1.8 Hz, 1H), 7.32 (dd, J = 7.6, 4.8 Hz, 1H), 2.30 (s, 3H).

### Example 8: Synthesis of Compound 8

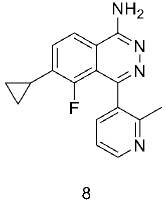

The synthetic route for Compound 8 is shown below: 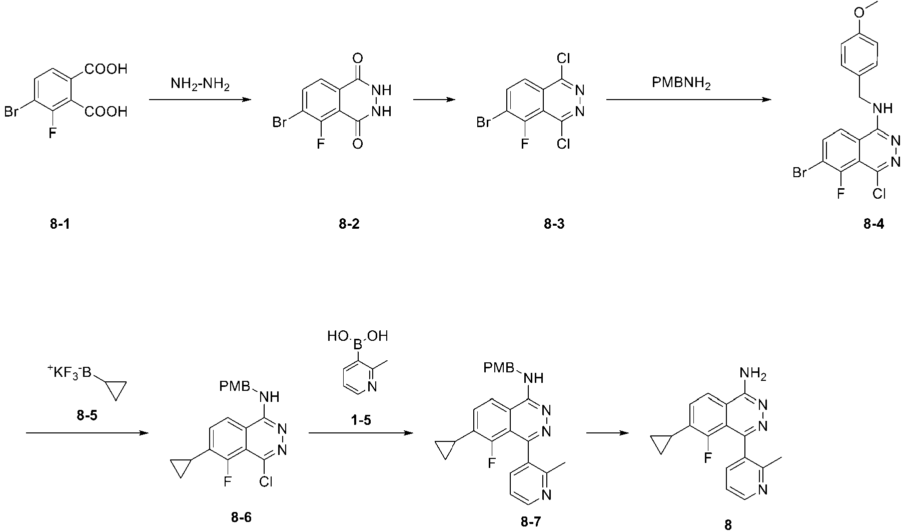
(1) Compound 8-1 (2 g, 7.60 mmol) was added to thionyl chloride (20 mL), and two drops of dry N,N-dimethylformamide were added. The mixture was heated to reflux for 2 hours and concentrated under reduced pressure. The residue was dissolved in acetic acid (30 mL); anhydrous hydrazine (1.52 g, 30.41 mmol) was then added, and the mixture was heated to reflux at 120 °C overnight. The reaction mixture was concentrated to dryness directly, water (20 mL) was added, and a solid precipitated. The mixture was filtered, and the filter cake was dried in vacuo overnight to afford Compound 8-2. MS-ESI [M+H]⁺, calc. 259, found 259.
(2) Compound 8-2 (3 g, 13.03 mmol) was dissolved in phosphorus oxychloride (30 mL), and the mixture was stirred at 100 °C for 2 hours. The reaction mixture was concentrated under reduced pressure to remove phosphorus oxychloride, and the resulting crude oil was slowly added dropwise to ice water (50 mL) with thorough stirring, adjusted to pH = 8 with 2 mol/L aqueous sodium hydroxide solution, and extracted with ethyl acetate (50 mL × 3). The combined organic layers were washed with saturated brine (50 mL × 3), dried over anhydrous sodium sulfate, and filtered, and the filtrate was concentrated under reduced pressure to remove the solvent. Purification by silica gel column chromatography (petroleum ether : ethyl acetate = 10:1) afforded Compound 8-3. MS-ESI [M+H]⁺, calc. 295, found 295.
(3) To a solution of Compound 8-3 (700 mg, 2.37 mmol) in dimethyl sulfoxide (10 mL) were added 4-methoxybenzylamine (324 mg, 2.37 mmol) and sodium carbonate (752 mg, 7.10 mmol). The mixture was stirred at 130 °C overnight. The reaction mixture was poured into water and extracted with ethyl acetate; the organic phase was washed with saturated brine, dried, and concentrated. The crude product was purified by silica gel column chromatography (petroleum ether : ethyl acetate = 3:1) to afford Compound 8-4. MS-ESI [M+H]⁺, calc. 396, found 396
(4) Compound 8-4 (230 mg, 0.58 mmol) was dissolved in 1,4-dioxane (5 mL); under nitrogen protection, potassium cyclopropyltrifluoroborate (103 mg, 0.70 mmol), sodium carbonate (184 mg, 1.74 mmol), and 1,1'-bis(dicyclohexylphosphino)ferrocene-palladium(II) dichloride (88 mg, 0.13 mmol) were added. The mixture was stirred under microwave irradiation at 90 °C for 3 hours under nitrogen protection, and the reaction was monitored to completion by LCMS. After concentration of the reaction mixture, the crude product was purified by silica gel column chromatography (petroleum ether : ethyl acetate = 2:1) to afford Compound 8-6. MS-ESI [M+H]⁺, calc. 358, found 358.
(5) To a solution of Compound 8-6 (80 mg, 0.22 mmol) in 1,4-dioxane (2 mL) and water (0.4 mL) were added Compound 1-5 (61.16 mg, 0.45 mmol), sodium carbonate (71 mg, 0.67 mmol), and bis(diphenylphosphino)ferrocene-palladium(II) dichloride (36.6 mg, 0.04 mmol). The mixture was stirred at 110 °C overnight under nitrogen protection. After concentration of the reaction mixture, the crude product was purified by silica gel column chromatography (methanol/dichloromethane = 2%-3%) to afford Compound 8-7. MS-ESI [M+H]⁺, calc. 415, found 415.
(6) At 0 °C, Compound 8-7 (50 mg, 0.12 mmol) was dissolved in a mixed solution of trifluoroacetic acid (1 mL) and trifluoromethanesulfonic acid (0.2 mL). The mixture was stirred for an additional 60 minutes under nitrogen protection, and the reaction was monitored to completion by LCMS. The reaction mixture was concentrated, and the crude product was purified by preparative high-performance liquid chromatography (Waters-CORTECS-C18-2.7 µm-4.6 × 30 mm; 25 mL/min; 25 °C; 0%-100% acetonitrile/0.05% NH4HCO3 aqueous buffer) to afford Compound 8. MS-ESI [M+H]⁺, calc. 295, found 295. ¹H NMR (400 MHz, DMSO-d6) δ 8.52 (dd, J = 4.8, 1.6 Hz, 1H), 8.11 (d, J = 8.4 Hz, 1H), 7.67 (dd, J = 7.6, 1.6 Hz, 1H), 7.49 (dd, J = 8.4, 7.2 Hz, 1H), 7.39-7.30 (m, 3H), 2.20 (s, 3H), 2.13 (td, J = 8.4, 4.0 Hz, 1H), 1.08-1.02 (m, 2H), 0.94-0.82 (m, 2H).

### Example 9: Synthesis of Compound 9

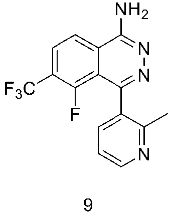

The synthetic route for Compound 9 is shown below: 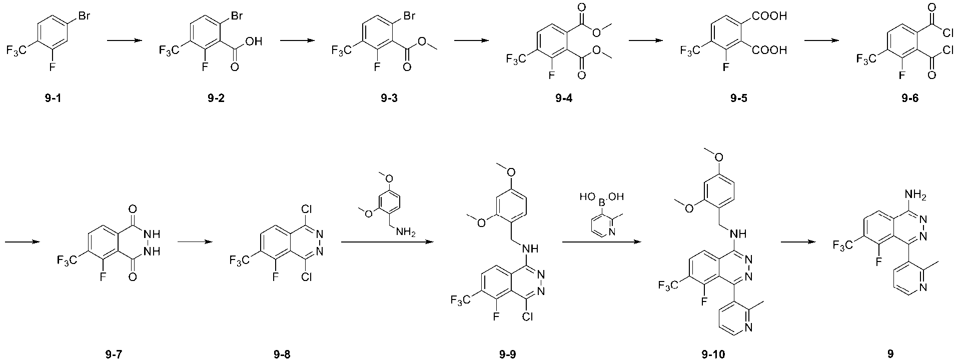
(1) At -70 °C, lithium diisopropylamide (2 mol/L, 30.86 mL, 61.73 mmol) was added dropwise to tetrahydrofuran (70 mL), and Compound 9-1 (10 g, 41.15 mmol) was then slowly added dropwise to the reaction mixture over approximately 20 minutes. After gradual warming to room temperature with stirring for 1.5 hours, carbon dioxide gas was bubbled into the reaction mixture, which was then stirred for an additional 1 hour. The reaction was quenched with 3 mol/L hydrochloric acid, and the solution pH was adjusted to 1-2. The mixture was then extracted with ethyl acetate; the combined organic layers were dried over anhydrous sodium sulfate and filtered, and the filtrate was concentrated under reduced pressure to remove the solvent, affording Compound 9-2.
(2) Compound 9-2 (8 g, 27.87 mmol) and potassium carbonate (5.78 g, 41.81 mmol) were dissolved in N,N-dimethylformamide (60 mL). At 0 °C, iodomethane (2.49 mL, 30.66 mmol) was added dropwise to the reaction mixture, which was then stirred at 25 °C for 12 hours. The reaction mixture was poured into water and extracted with ethyl acetate; the combined organic layers were washed with saturated brine, dried over anhydrous sodium sulfate, and filtered, and the filtrate was concentrated under reduced pressure to remove the solvent. Purification by column chromatography (petroleum ether : ethyl acetate = 50:1) afforded Compound 9-3 as a colorless oil.
(3) Compound 9-3 (8 g, 26.58 mmol) and N,N-diisopropylethylamine (22.02 mL, 132.88 mmol) were dissolved in methanol (40 mL) and N,N-dimethylformamide (40 mL); palladium acetate (298.32 mg, 1.33 mmol) and 4,5-bis(diphenylphosphino)-9,9-dimethylxanthene (1.54 g, 2.66 mmol) were then added to the reaction mixture. The reaction mixture was then stirred at 80 °C for 16 hours under a carbon monoxide atmosphere. Water was added to the reaction mixture, which was extracted with ethyl acetate; the combined organic layers were washed with saturated brine, dried over anhydrous sodium sulfate, and filtered, and the filtrate was concentrated under reduced pressure to remove the solvent. Purification by column chromatography (petroleum ether : ethyl acetate = 10:1) afforded Compound 9-4.
(4) Compound 9-4 (3.0 g, 10.71 mmol) was dissolved in tetrahydrofuran (15 mL) and water (15 mL). At 0 °C, solid sodium hydroxide (1.285 g, 32.12 mmol) was added to the reaction mixture, which was then stirred at 25 °C for 3 hours. The reaction mixture was quenched with 3 mol/L hydrochloric acid at 0-10 °C, and the solution pH was adjusted to 1-2. The mixture was then extracted with ethyl acetate; the combined organic layers were dried over anhydrous sodium sulfate and filtered, and the filtrate was concentrated under reduced pressure to remove the solvent, affording Compound 9-5.
(5) Compound 9-5 (2.3 g, 9.12 mmol) was dissolved in thionyl chloride (20 mL), and two drops of dry N,N-dimethylformamide were then added. The mixture was heated to reflux at 80 °C for 2 hours under nitrogen protection. The reaction mixture was concentrated to dryness, and the residue was redissolved and concentrated three times with toluene (20 mL) to afford crude Compound 9-6.
(6) Compound 9-6 (2.8 g, 9.69 mmol) was dissolved in acetic acid (25 mL); hydrazine hydrate (2.85 g, 48.44 mmol) was added, and the mixture was heated to reflux at 120 °C for 12 hours. The reaction mixture was concentrated to dryness directly, water was added to disperse the solid, and the mixture was filtered. The filter cake was suction-dried and then dried in vacuo overnight to afford Compound 9-7 as a pale yellow solid. MS-ESI [M+H]⁺, calc. 249, found 249.
(7) Compound 9-7 (2.3 g, 9.27 mmol) was dissolved in phosphorus oxychloride (20 mL) and stirred at 100 °C for 2 hours. The reaction mixture was concentrated directly to remove the phosphorus oxychloride. The oily residue was redissolved in ethyl acetate, and the solution was slowly added dropwise to aqueous sodium bicarbonate solution with thorough stirring. The mixture was extracted with ethyl acetate; the combined organic layers were washed with saturated brine, dried over anhydrous sodium sulfate, and filtered, and the filtrate was concentrated under reduced pressure to remove the solvent. Purification by column chromatography (petroleum ether : ethyl acetate = 8:1) afforded Compound 9-8 as an off-white solid. MS-ESI [M+H]⁺, calc. 285, found 285.
(8) Compound 9-8 (700 mg, 2.46 mmol) was dissolved in N-methylpyrrolidone (7 mL); (2,4-dimethoxyphenyl)methylamine (410.6 mg, 2.46 mmol) and N,N-diisopropylethylamine (634.8 mg, 4.91 mmol) were then added, and the mixture was stirred at 130 °C for 2 hours. The reaction mixture was poured into water and extracted with ethyl acetate; the combined organic layers were washed with saturated brine, dried, and concentrated. Purification by silica gel column chromatography (petroleum ether : ethyl acetate = 3:1) afforded Compound 9-9. MS-ESI [M+H]⁺, calc. 416, found 416.
(9) Compound 9-9 (270 mg, 0.65 mmol) was dissolved in 1,4-dioxane (10 mL) and water (1 mL); under nitrogen protection, 2-methylpyridine-3-boronic acid (177.8 mg, 1.30 mmol), sodium bicarbonate (136.4 mg, 1.62 mmol), and 1,1'-bis(diphenylphosphino)ferrocene-palladium(II) dichloride (53.2 mg, 0.06 mmol) were added. The mixture was stirred at 105 °C for 3 hours under nitrogen protection. The reaction mixture was poured into water and extracted with ethyl acetate; the combined organic layers were washed with saturated brine, dried, and concentrated. Purification by silica gel column chromatography (petroleum ether : ethyl acetate = 3:1) afforded Compound 9-10. MS-ESI [M+H]⁺, calc. 473, found 473.
(10) Compound 9-10 (160 mg, 0.34 mmol) was dissolved in dichloromethane (1 mL); then at 0 °C, trifluoroacetic acid (1 mL) and trifluoromethanesulfonic acid (0.2 mL) were added dropwise to the reaction mixture. The reaction was stirred at 25 °C for 1 hour. The reaction mixture was concentrated directly, and the oily residue was redissolved in dichloromethane. The solution was then slowly added dropwise to ice-cold aqueous sodium bicarbonate solution with thorough stirring, the aqueous phase was adjusted to pH = 8, and the mixture was extracted with dichloromethane. The combined organic layers were washed with saturated brine, dried over anhydrous sodium sulfate, and filtered, and the filtrate was concentrated under reduced pressure to remove the solvent. Purification by column chromatography (dichloromethane : methanol = 10:1) afforded Compound 9. MS-ESI [M+H]⁺, calc. 323, found 323. ¹H NMR (400 MHz, DMSO-d6) δ 8.55 (dd, J = 1.6, 4.8 Hz, 1H), 8.39 (d, J = 8.8 Hz, 1H), 8.30-8.24 (m, 1H), 7.73 (dd, J = 1.6, 7.6 Hz, 1H), 7.62 (s, 2H), 7.35 (dd, J = 5.2, 7.6 Hz, 1H), 2.21 (s, 3H).

### Example 10: Synthesis of Compound 10

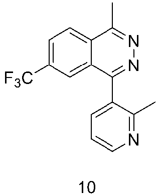

The synthetic route for Compound 10 is shown below: 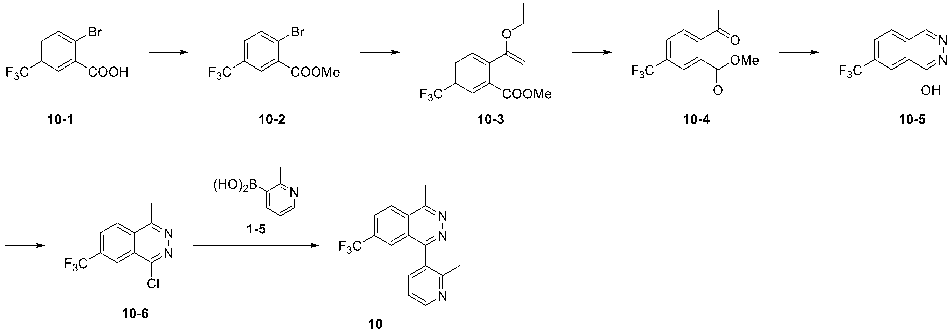
(1) Compound 10-1 (3.0 g, 11.15 mmol) was dissolved in methanol (30 mL), and thionyl chloride (1.21 mL, 16.73 mmol) was added. The mixture was heated to 70 °C and stirred for 2 hours under a nitrogen atmosphere. The reaction was monitored to completion by LCMS. The reaction mixture was concentrated, and the crude product was purified by silica gel column chromatography (3%-5% ethyl acetate/petroleum ether) to afford Compound 10-2.
(2) Compound 10-2 (1.50 g, 5.30 mmol) and tributyl(1-ethoxyvinyl)tin (2.69 mL, 7.95 mmol) were dissolved in 1,4-dioxane (25 mL); under a nitrogen atmosphere, tetrakis(triphenylphosphine)palladium (1.23 g, 1.06 mmol) was added, and the mixture was stirred at 100 °C for 12 hours. The reaction was monitored by LCMS. The reaction mixture was poured into water and extracted with ethyl acetate; the combined organic layers were washed with saturated brine, dried, and concentrated to afford Compound 10-3. MS-ESI [M+H]⁺, calc. 275, found 275. ¹H NMR (400 MHz, CDCl3) δ 7.87-7.81 (m, 1H), 7.64-7.59 (m, 1H), 7.52 (d, J = 8.4 Hz, 1H), 4.37 (d, J = 2.8 Hz, 1H), 4.27 (d, J = 2.8 Hz, 1H), 3.84-3.79 (m, 5H), 1.25 (d, J = 7.2 Hz, 3H).
(3) At room temperature, Compound 10-3 (998 mg, 2.55 mmol) was dissolved in a solution of hydrochloric acid in 1,4-dioxane (4 mol/L, 10 mL) and stirred at 25 °C for 2 hours. The reaction was monitored to completion by LCMS. After concentration of the reaction mixture, crude Compound 10-4 was obtained. MS-ESI [M+H]⁺, calc. 247, found 247.
(4) Compound 10-4 (1.0 g, 4.07 mmol) and hydrazine hydrate (1 mL, 20.58 mmol) were dissolved in ethanol (10 mL) and heated to reflux at 90 °C for 2 hours under a nitrogen atmosphere. The reaction was monitored by LCMS. After concentration of the reaction mixture, the crude product was purified by silica gel column chromatography with a gradient of methanol/dichloromethane = 2%-3% to afford Compound 10-5. MS-ESI [M+H]⁺, calc. 229, found 229. ¹H NMR (400 MHz, CDCl3) δ 10.26 (s, 1H), 8.73-8.67 (m, 1H), 8.00 (dd, J = 8.4, 1.8 Hz, 1H), 7.86 (d, J = 8.4 Hz, 1H), 2.57 (s, 3H).
(5) Compound 10-5 (551 mg, 2.41 mmol) was dissolved in phosphorus oxychloride (7 mL) and heated to reflux at 110 °C for 1 hour under a nitrogen atmosphere. The reaction was monitored by LCMS. After concentration of the reaction mixture, the crude product was purified by silica gel column chromatography with a gradient of methanol/dichloromethane = 5%-7% to afford Compound 10-6. MS-ESI [M+H]⁺, calc. 247, found 247. ¹H NMR (400 MHz, CDCl3) δ 8.54 (s, 1H), 8.18 (d, J = 8.4 Hz, 1H), 8.11 (dd, J = 8.4, 1.6 Hz, 1H), 2.98 (s, 3H).
(6) Compound 10-6 (100 mg, 0.41 mmol) was dissolved in 1,4-dioxane (5 mL) and water (1 mL); under nitrogen protection, 2-methylpyridine-3-boronic acid (72.18 mg, 0.53 mmol), sodium carbonate (107.44 mg, 1.01 mmol), and 1,1'-bis(diphenylphosphino)ferrocene-palladium(II) dichloride (60.16 mg, 0.06 mmol) were added. The mixture was stirred at 100 °C overnight under nitrogen protection, and the reaction was monitored to completion by LCMS. After concentration of the reaction mixture, the crude product was purified by silica gel column chromatography with a gradient of methanol/dichloromethane = 5%-7% to afford a crude compound. The crude compound was further purified by reverse-phase column chromatography (Waters-CORTECS-C18-2.7 µm-4.6 × 30 mm; 25 mL/min; 25 °C; 0%-100% acetonitrile/0.05% formic acid aqueous buffer) to afford Compound 10. MS-ESI [M+H]⁺, calc. 304, found 304. ¹H NMR (400 MHz, CDCl3) δ 8.82-8.64 (m, 1H), 8.35 (d, J = 8.4 Hz, 1H), 8.14 (d, J = 8.4 Hz, 1H), 7.85 (s, 1H), 7.72 (d, J = 7.2 Hz, 1H), 7.38 (t, J = 5.6 Hz, 1H), 3.16 (s, 3H), 2.37 (s, 3H).

### Example 11: Synthesis of Compound 11

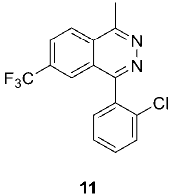

The synthetic route for Compound 11 is shown below:

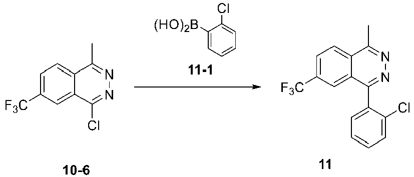

Compound 10-6 (100 mg, 0.41 mmol) was dissolved in 1,4-dioxane (5 mL) and water (1 mL); under nitrogen protection, 2-chlorophenylboronic acid (83.35 mg, 0.53 mmol), sodium carbonate (107.44 mg, 1.01 mmol), and 1,1'-bis(diphenylphosphino)ferrocene-palladium(II) dichloride (60.16 mg, 0.06 mmol) were added. The mixture was stirred at 100 °C overnight under nitrogen protection, and the reaction was monitored to completion by LCMS. After concentration of the reaction mixture, the crude product was purified by silica gel column chromatography with a gradient of methanol : dichloromethane = 1%-2% to afford a crude compound. The crude compound was further purified by reverse-phase column chromatography (Waters-CORTECS-C18-2.7 µm-4.6 × 30 mm; 25 mL/min; 25 °C; 0%-100% acetonitrile/0.05% NH4HCO3 aqueous buffer) to afford Product 11. MS-ESI [M+H]⁺, calc. 323, found 323. ¹H NMR (400 MHz, CDCl3) δ 8.31 (d, J = 8.8 Hz, 1H), 8.11 (dd, J = 8.8, 1.6 Hz, 1H), 7.92-7.86 (m, 1H), 7.63-7.48 (m, 4H), 3.15 (s, 3H).

### Biological Experimental Example 1

**Experimental Principle:** MAT2A catalyzes the reaction of L-methionine and ATP to produce S-adenosyl methionine (SAM) and phosphate. The phosphate can be detected by formation of malachite green phosphomolybdate with sulfuric acid and ammonium molybdate.

**Experimental Materials:** MAT2A enzyme was purchased from BPS (Cat. No. 71401-1); tris(hydroxymethyl)aminomethane (Tris) was purchased from Life Science (Cat. No. 0497); bovine serum albumin (BSA) was purchased from Sigma (Cat. No. V900933); L-methionine was purchased from Adams (Cat. No. 1100469); ATP was purchased from Sigma (Cat. No. A7699); magnesium chloride was purchased from Sigma (Cat. No. M8266); potassium chloride was purchased from Sigma (Cat. No. 7447-40-7); polyoxyethylene lauryl ether (Brij35) was purchased from Sigma (Cat. No. B4184); ethylenediaminetetraacetic acid (EDTA) was purchased from Sigma (Cat. No. M1644); dimethyl sulfoxide (DMSO) was purchased from Sigma (Cat. No. D2650); BIOMOL^{®} Green was purchased from Enzo (Cat. No. BML-AK111-0250); 384-well plates were purchased from Corning (Cat. No. 3765); the centrifuge was purchased from Eppendorf (Model 5430); the microplate reader was purchased from BioTek (Model Synergy 2); the pipettor was purchased from Labcyte (Model Echo 550).

**Experimental Method:** A buffer was prepared with the following components: 50 mM Tris, pH 7.5 / 50 mM potassium chloride / 10 mM magnesium chloride / 0.01% Brij-35 / 1 mM EDTA / 0.1% BSA. The final concentration of test compounds started at 10 µM, with 3 -fold serial dilution over 10 concentrations, tested in duplicate wells. In a 384-well plate, gradient dilutions were prepared as 10 solutions at 100× final concentration. Then 250 nL was transferred using an Echo 550 to a 384-well reaction plate for subsequent use. Negative control wells and positive control wells received 250 nL of 100% DMSO each. A 1.67× final concentration MAT2A enzyme solution was prepared in buffer. To the compound wells and positive control wells, 15 µL of the 1.67× final concentration MAT2A enzyme solution was added; to the negative control wells, 15 µL of buffer was added. The plate was centrifuged at 1000 rpm for 60 seconds, mixed by shaking, and incubated for 15 minutes. A 2.5× final concentration substrate mixture solution was prepared in buffer (substrates: L-methionine and ATP). 10 µL of the 2.5× final concentration substrate mixture was added to initiate the reaction. The 384-well plate was centrifuged at 1000 rpm for 60 seconds, mixed by shaking, and incubated for 150 minutes. 50 µL of BIOMOL^{®} Green was added to terminate the reaction, and after centrifugation at 1000 rpm for 60 seconds, the plate was incubated for 15 minutes. OD620 was read and the data analyzed.

### Experimental Data:

The specific test results are shown in Table 1:

**Table 1: MAT2A IC₅₀ data for selected compounds**

| Test Compound | MAT2A IC₅₀ (nM) |
|---|---|
| Compound 1 | A |
| Compound 4 | A |
| Compound 9 | A |

A indicates 0 < IC₅₀ < 200 nM; B indicates 200 nM ≤ IC₅₀ < 1000 nM; C indicates 1000 nM ≤ IC₅₀ < 10000 nM; D indicates IC₅₀ ≥ 10000 nM.

As can be seen from the test data in Table 1, the fused bicyclic compounds of Formula I according to the present invention exhibit favorable inhibitory activity against MAT2A and have potential for use in the preparation of medicaments for the prevention or treatment of MTAP^{-/-} associated cancers.

### Biological Experimental Example 2

### Proliferation-Inhibitory Activity Against HCT116 MTAP^{-/-} and HCT116 MTAP^{WT} Cells

**Experimental Principle:** HCT116 MTAP^{-/-} cells lack MTAP and exhibit elevated MTA levels; HCT116 MTAP^{wt} cells retain MTAP and show normal MTA levels. These two cell lines are used to determine whether compounds display MTA-synergistic inhibitory activity.

**Experimental Materials:** HCT116 MTAP^{-/-} and HCT116 MTAP^{WT} cells were purchased from Horizon; CellTiter-Glo reagent was purchased from Promega (Cat. No. G7571); RPMI-1640 was purchased from ATCC (Cat. No. 30-2001); fetal bovine serum (FBS) was purchased from EXCELL (Cat. No. FND500); penicillin-streptomycin was purchased from Gibco (Cat. No. 15140-122); 0.25% trypsin-ethylenediaminetetraacetic acid digestion solution (Trypsin-EDTA) was purchased from Gibco (Cat. No. 25200-072); dimethyl sulfoxide (DMSO) was purchased from Sigma (Cat. No. D2650); 96-well plates were purchased from Corning (Cat. No. 3610); the incubator was purchased from NuAire (Model NU-5700E); the inverted microscope was purchased from Nikon (Model TS-100); the automated cell counter was purchased from Life Technologies (Model Countess II); the microplate reader was purchased from PerkinElmer (Model Envision); the data analysis software was GraphPad Prism 5.0.

**Experimental Method:** HCT116 MTAP^{-/-} or HCT116 MTAP^{WT} cells in logarithmic growth phase were resuspended in growth medium (RPMI-1640 + 10% FBS) and diluted to the target density (5000/mL). The cell suspension was seeded into 96-well plates at 100 µL per well, and the plates were incubated overnight at 37 °C in a 5% CO₂ incubator. Medium alone served as the background control group. The cells were starved with 80 µL of serum-free medium for 4 hours. The test compounds were dissolved in DMSO to prepare a 10 mmol/L stock solution. The stock solution was first diluted to 2 mmol/L (200×) with DMSO, then serially diluted 3-fold over a total of 10 concentrations. From each concentration, 3 µL of the above solution was diluted with 297 µL of growth medium (2×). Then 80 µL/well was added to the 96-well plates containing seeded cells. The cells with added test compounds were incubated at 37 °C in a 5% CO2 incubator for 120 hours. The 96-well plates were equilibrated at room temperature, and 40 µL of CellTiter-Glo reagent was added to each well, mixed on a vortex for 2 minutes, and incubated at room temperature for 60 minutes. The luminescence was read on an EnVision microplate reader, and the IC₅₀ values of the compounds were calculated using GraphPad Prism 5.0 software. The results are shown in Table 2.

**Table 2**

| Compound | | IC₅₀ (nM) | |
|---|---|---|---|
| | | HCT116 MTAP^{-/-} | HCT116 MTAP^{WT} |
| Compound | 1 | B | ND |
| Compound | 3 | B | ND |
| Compound | 4 | A | D |
| Compound | 6 | B | ND |
| Compound | 8 | B | ND |
| Compound | 9 | A | ND |

A indicates 0 < IC₅₀ < 100 nM; B indicates 100 nM ≤ IC₅₀ < 1000 nM; C indicates 1000 nM ≤ IC₅₀ < 10000 nM; D indicates IC₅₀ ≥ 10000 nM; ND indicates not detected.

### Biological Example 3

### Proliferation Inhibition of Various MTAP Wild-Type or Deleted Tumor Cells

**Experimental Principle:** The human tumor cell lines HCC15, NCI-H2052, LN18, NCI-H838, LU99, DOHH2, HT-1080, MKN45, THP-1, MCF7, MIA PaCa-2, U87-MG, and UMUC3 are all MTAP gene-deleted cell lines. NCI-H1944, ASPC-1, U251, and HT-1376 are MTAP gene wild-type cell lines. In MTAP-deleted cell lines, the compounds of the present invention are able to inhibit the proliferation of such MTAP-deleted cells via binding to MAT2A, while exhibiting poor proliferation-inhibitory activity against MTAP wild-type cells.

**Experimental Materials:** Information regarding the cell lines used in the above experiments is detailed in Table 3; CellTiter-Glo^{®} was purchased from Promega (Cat. No. G7571); Luminescent Cell Viability Detection Kit was purchased from VEKY-BIO (Cat. No. A2010003N); RPMI-1640 was purchased from Viva Cell (Cat. No. C3010-0500); DMEM was purchased from Viva Cell (Cat. No. C3113-0500); EMEM was purchased from Wisent (Cat. No. 320-005-CL); fetal bovine serum (FBS) was purchased from Biosera (Cat. No. FB-1058/500); horse serum was purchased from Gibco (Cat. No. 16050-122); NEAA was purchased from Wisent (Cat. No. 321-011-EL); penicillin-streptomycin was purchased from BasalMedia (Cat. No. S110JV); sodium pyruvate was purchased from Macklin (Cat. No. P6033); 0.25% trypsin-ethylenediaminetetraacetic acid digestion solution (Trypsin-EDTA) was purchased from BasalMedia (Cat. No. S310KJ); dimethyl sulfoxide (DMSO) was purchased from Sinopharm (Cat. No. 30072418); 96-well plates for 2D culture (used only for the NCI-H838 cell line) were purchased from Corning (Cat. No. 3610); 96-well plates for 3D culture (used for all cell lines except NCI-H838 ) were purchased from Corning (Cat. No. 3707); the incubator was purchased from PHCbi (Model MCO-18AC); the inverted microscope was purchased from Mingmei (Cat. No. MI52-0); the automated cell counter was purchased from BodBoge (Model JSY-SC-031N); the microplate reader was purchased from PerkinElmer (Model Envision); the data analysis software was GraphPad Prism 8.0.

**Experimental Method:** Each of the above cell lines in logarithmic growth phase was resuspended in growth medium and diluted to an appropriate density (approximately 6250/mL). The cell suspensions were seeded into 96-well plates at 80 µL per well, and the plates were incubated overnight at 37 °C in a 5% CO2 incubator. Medium alone served as the background control group. The test compounds were dissolved in DMSO to prepare a 10 mmol/L stock solution. The stock solution was first diluted to 2 mmol/L with DMSO, then serially diluted 5-fold over a total of 9 concentrations. From each concentration, 2 µL of the above solution was diluted with 78 µL of growth medium. Then 20 µL/well was added to the 96-well plates containing seeded cells. The cells with added test compounds were incubated at 37 °C in a 5% CO2 incubator with medium change at day 6, and continued incubation until day 10. The 96-well plates were equilibrated at room temperature, and 100 µL of detection reagent (Luminescent Cell Viability Detection) or 25 µL of CellTiter-Glo reagent was added to each well; after incubation at room temperature for 10 minutes, the luminescence was read on an EnVision microplate reader, and the IC₅₀ values of the compounds were calculated using GraphPad Prism 8.0 software. The specific test results are shown in Table 3.

**Table 3: Cell Line Names, Cancer Types and Culture Media**

| Cell Line | Cancer Type | Culture Medium |
|---|---|---|
| HCC15 | NSCLC | RPMI-1640+10%FBS |
| NCI-H2052 | MESO | RPMI-1640+10%FBS |
| LN18 | GBM | DMEM+10%FBS |
| NCI-H838 | LUAD | RPMI-1640+10%FBS |
| LU99 | LCLC | RPMI-1640+10%FBS |
| DOHH2 | BCL | RPMI-1640+10%FBS |
| HT-1080 | SARC | EMEM + 10% FBS+ 1% NEAA + 1mM Sodium Pyruvate (NaP) |
| MKN45 | STAD | RPMI-1640+10%FBS |
| THP-1 | LAML | RPMI-1640+10%FBS |
| MCF7 | BRCA | EMEM+1%NEAA + 10% FBS |
| MIA PaCa-2 | PDAC | DMEM+10%FBS+5% horse serum |
| U87-MG | GBM | EMEM + 1% NEAA + 1mM Sodium Pyruvate (NaP) + 10% FBS |
| UMUC3 | BLCA | EMEM + 10% FBS+ 1% NEAA + 1mM Sodium Pyruvate (NaP) |
| NCI-H1944 | LUAD | RPMI-1640+10%FBS |
| ASPC-1 | PDAC | RPMI-1640+10%FBS |
| U251 | GBM | EMEM + 10% FBS + 1% NEAA + 1mM Sodium Pyruvate (NaP) |
| HT-1376 | BLCA | EMEM+ 10% FBS + 1% NEAA + 1mM Sodium Pyruvate (NaP) |

**Table 4: Proliferation Activity of Compound 4 on Cell Lines of Various MTAP Genotypes**

| Cell Line | MTAP Genotypes | IC₅₀ |
|---|---|---|
| HCC15 | Deletion | B |
| NCI-H2052 | Deletion | A |
| LN18 | Deletion | B |
| NCI-H838 | Deletion | A |
| LU99 | Deletion | B |
| DOHH2 | Deletion | B |
| HT-1080 | Deletion | B |
| MKN45 | Deletion | B |
| THP-1 | Deletion | B |
| MCF7 | Deletion | B |
| Mia Paca2 | Deletion | C |
| U87MG | Deletion | C |
| UMUC3 | Deletion | N.T. |
| NCI-H1944 | WT | C |
| ASPC-1 | WT | D |
| U251 | WT | D |
| HT-1376 | WT | N.T. |

A indicates 0 < IC₅₀ < 100 nM; B indicates 100 nM ≤ IC₅₀ < 1000 nM; C indicates 1000 nM ≤ IC₅₀ < 10000 nM; D indicates IC₅₀ ≥ 10000 nM; ND indicates not detected; N.T. indicates not tested.

The Applicant declares that the present invention illustrates the fused bicyclic compounds, the pharmaceutical compositions comprising them, and their use through the above-described examples; however, the present invention is not limited to the above-described examples, i.e., it does not mean that the present invention must rely on the above-described examples for its implementation. All references mentioned in the present invention are incorporated by reference into this application, as if each reference were individually incorporated by reference. Those skilled in the art should understand that any improvement to the present invention, any equivalent substitution of the various raw materials and addition of auxiliary components in the products of the present invention, the selection of specific embodiments, and the like, all fall within the scope of protection and disclosure of the present invention.

## Claims

1. A compound of Formula I, or a pharmaceutically acceptable salt, enantiomer, diastereomer, tautomer, cis-trans isomer, solvate, polymorph, deuterated form thereof, or a combination thereof, 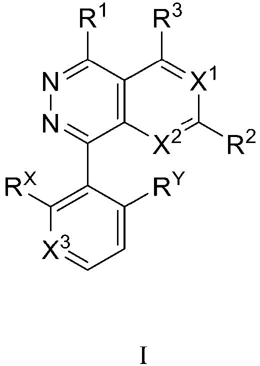 wherein,
R¹ is selected from the group consisting of: hydrogen, hydroxy, optionally substituted C₁-C₆ alkyl, optionally substituted C₂-C₆ alkenyl, optionally substituted C₂-C₆ alkynyl, optionally substituted 3- to 10-membered carbocyclyl, optionally substituted 4- to 8-membered heterocyclyl, optionally substituted 6- to 10-membered aryl, optionally substituted 5- to 10-membered heteroaryl, -L¹-R⁴, and -NR⁵R⁶; in R¹, said "optionally substituted" means that the specified group is unsubstituted or is substituted by one or more R^{1s}, and each R^{1s} is independently selected from the group consisting of: halo, hydroxy, amino, cyano, oxo, C₁-C₆ alkyl, C₁-C₆ haloalkyl, C₂-C₄ alkenyl, C₂-C₄ haloalkenyl, C₂-C₄ alkynyl, C₂-C₄ haloalkynyl, C₁-C₆ alkoxy-C₁-C₆ alkyl, hydroxy-C₁-C₆ alkyl-, C₁-C₆ alkylamino, C₁-C₆ alkoxy, C₁-C₆ haloalkoxy, 3- to 10-membered carbocyclyl, 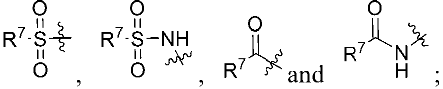
L¹ is selected from the group consisting of: -O-, -S-, -SO-, -SO₂-, and -CO-;
R⁴ is selected from the group consisting of: optionally substituted C₁-C₆ alkyl, optionally substituted 3- to 10-membered carbocyclyl, optionally substituted 4- to 8-membered heterocyclyl, optionally substituted 6- to 10-membered aryl, and optionally substituted 5- to 10-membered heteroaryl; in R⁴, said "optionally substituted" means that the specified group is unsubstituted or is substituted by one or more R^{4s}, and each R^{4s} is independently selected from the group consisting of: halo, hydroxy, amino, cyano, oxo, C₁-C₆ alkoxy, C₁-C₆ haloalkoxy, C₂-C₄ alkenyl, C₂-C₄ haloalkenyl, C₂-C₄ alkynyl, C₂-C₄ haloalkynyl, C₁-C₆ alkoxy-C₁-C₆ alkyl, hydroxy-C₁-C₆ alkyl, amino-C₁-C₆ alkyl, 3- to 10-membered carbocyclyl, 3- to 10-membered carbocyclyloxy, 4- to 8-membered heterocyclyl, 4- to 8-membered heterocyclyloxy, 6- to 10-membered aryl, 5- to 10-membered heteroaryl, 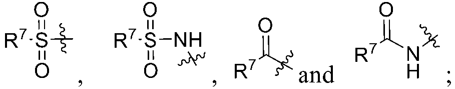
R⁵ is selected from the group consisting of: hydrogen, C₁-C₆ alkyl, C₁-C₆ haloalkyl, and 3- to 10-membered carbocyclyl;
R⁶ is selected from the group consisting of: hydrogen, optionally substituted C₁-C₆ alkyl, optionally substituted 3- to 10-membered carbocyclyl, optionally substituted 4- to 10-membered heterocyclyl, optionally substituted 6- to 10-membered aryl, optionally substituted 5- to 10-membered heteroaryl, 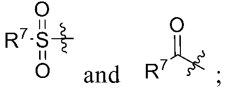 in R⁶, said "optionally substituted" means that the specified group is unsubstituted or is substituted by one or more R^{6s}, and each R^{6s} is independently selected from the group consisting of: deuterium, halo, hydroxy, amino, cyano, C₁-C₆ alkyl, C₁-C₆ haloalkyl, hydroxy-C₁-C₆ alkyl, C₁-C₆ alkoxy, C₁-C₆ haloalkoxy, C₂-C₄ alkenyl, C₂-C₄ haloalkenyl, C₂-C₄ alkynyl, C₂-C₄ haloalkynyl, 3- to 10-membered carbocyclyl optionally substituted by one or more R8, 3- to 10-membered carbocyclyloxy optionally substituted by one or more R⁸, 4- to 8-membered heterocyclyl optionally substituted by one or more R⁸, 4- to 8-membered heterocyclyloxy optionally substituted by one or more R⁸, C₁-C₆ alkylamino, (C₁-C₆ alkyl)₂ amino, C₁-C₆ alkoxy-C₁-C₆ alkyl, amino-C₁-C₆ alkyl, 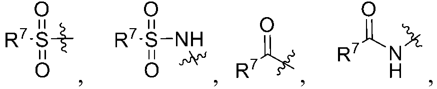 6- to 10-membered aryl optionally substituted by one or more R⁹, and 5- to 10-membered heteroaryl optionally substituted by one or more R⁹;
R² is selected from the group consisting of: halo, cyano, optionally substituted C₁-C₆ alkyl, optionally substituted C₂-C₆ alkenyl, optionally substituted C₂-C₆ alkynyl, optionally substituted 3- to 10-membered carbocyclyl, optionally substituted 4- to 8-membered heterocyclyl, -L²-R¹⁰, and -NR¹¹R¹²; in R², said "optionally substituted" means that the specified group is unsubstituted or is substituted by one or more R^{2s}, and each R^{2s} is independently selected from the group consisting of: halo, cyano, oxo, C₁-C₆ alkyl, C₁-C₆ haloalkyl, C₁-C₆ alkoxy, C₁-C₆ haloalkoxy, and 3- to 10-membered carbocyclyl;
R³ is selected from the group consisting of: hydrogen, halo, cyano, optionally substituted C₁-C₆ alkyl, optionally substituted C₂-C₆ alkenyl, optionally substituted C₂-C₆ alkynyl, optionally substituted 3- to 10-membered carbocyclyl, optionally substituted 4- to 8-membered heterocyclyl, -L^{2'}-R^{10'}, and -NR^{11'}R^{12'}; in R³, said "optionally substituted" means that the specified group is unsubstituted or is substituted by one or more R^{3s}, and each R^{3s} is independently selected from the group consisting of: halo, cyano, oxo, C₁-C₆ alkyl, C₁-C₆ haloalkyl, C₁-C₆ alkoxy, C₁-C₆ haloalkoxy, and 3- to 10-membered carbocyclyl;
L² and L^{2'} are each independently selected from the group consisting of: -O-, -S-, -SO-, -SO₂-, and -CO-;
R¹⁰ and R^{10'} are each independently selected from the group consisting of: optionally substituted C₁-C₆ alkyl, optionally substituted 3- to 10-membered carbocyclyl, optionally substituted 4- to 8-membered heterocyclyl, optionally substituted 6- to 10-membered aryl, and optionally substituted 5- to 10-membered heteroaryl; in R¹⁰ and R^{10'}, said "optionally substituted" means that the specified group is unsubstituted or is substituted by one or more R^{10s}, and each R^{10s} is independently selected from the group consisting of: halo, cyano, C₁-C₆ alkyl, C₁-C₆ haloalkyl, C₁-C₆ alkoxy, C₁-C₆ haloalkoxy, 3- to 10-membered carbocyclyl, 4- to 8-membered heterocyclyl, 6- to 10-membered aryl, and 5- to 10-membered heteroaryl;
R¹¹ and R^{11'} are each independently selected from the group consisting of: hydrogen and C₁-C₆ alkyl;
R¹² and R^{12'} are each independently selected from the group consisting of: optionally substituted C₁-C₆ alkyl, optionally substituted 3- to 10-membered carbocyclyl, optionally substituted 4- to 10-membered heterocyclyl, optionally substituted 6- to 10-membered aryl, optionally substituted 5- to 10-membered heteroaryl, 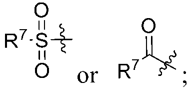 in R¹² and R^{12'}, said "optionally substituted" means that the specified group is unsubstituted or is substituted by one or more R^{12s}, and each R^{12s} is independently selected from the group consisting of: halo, cyano, C₁-C₆ alkyl, C₁-C₆ haloalkyl, C₁-C₆ alkoxy, C₁-C₆ haloalkoxy, 3- to 10-membered carbocyclyl, and 4- to 8-membered heterocyclyl;
X³ is selected from the group consisting of: CH and N;
R^{X} is selected from the group consisting of: methyl, ethyl, 3- to 4-membered carbocyclyl, halomethyl, or halo;
R^{Y} is selected from the group consisting of: hydrogen, methyl, halomethyl, or halo;
X¹ is selected from the group consisting of: CR¹³ and N;
X² is selected from the group consisting of: CR¹⁴ and N;
R¹³ and R¹⁴ are each independently selected from the group consisting of: hydrogen, halo, cyano, optionally substituted C₁-C₆ alkyl, optionally substituted C₁-C₆ alkoxy, optionally substituted 3- to 10-membered carbocyclyl, and optionally substituted 4- to 8-membered heterocyclyl; in R¹³ and R¹⁴, said "optionally substituted" means that the specified group is unsubstituted or is substituted by one or more R^{13s} or R^{14s}, and each R^{13s} and R^{14s} is independently selected from the group consisting of: halo and oxo (=O); with the proviso that when R^{X} and R² are both chloro and X¹ is CR¹³, R¹³ is not chloro;
Each R⁷ is independently selected from the group consisting of: C₁-C₆ alkyl, C₁-C₆ haloalkyl, C₁-C₆ alkoxy, C₁-C₆ haloalkoxy, 3- to 10-membered carbocyclyl, 4- to 8-membered heterocyclyl, amino, C₁-C₆ alkylamino, di(C₁-C₆ alkyl)amino, 6- to 10-membered aryl, and 5-to 10-membered heteroaryl;
Each R⁸ is independently selected from the group consisting of: hydrogen, halo, hydroxy, C₁-C₆ alkyl, C₁-C₆ haloalkyl, or hydroxy-C₁-C₆ alkyl;
Each R⁹ is independently selected from the group consisting of: hydrogen, halo, C₁-C₆ alkyl, or C₁-C₆ haloalkyl.

2. The compound according to claim 1, or a pharmaceutically acceptable salt, enantiomer, diastereomer, tautomer, cis-trans isomer, solvate, polymorph, deuterated form thereof, or a combination thereof, **characterized in that**:
R¹ is selected from the group consisting of: hydrogen, hydroxy, optionally substituted C₁-C₆ alkyl, optionally substituted 3- to 10-membered carbocyclyl, optionally substituted 4- to 8-membered heterocyclyl, optionally substituted 5- to 10-membered heteroaryl, -OR⁴, and -NR⁵R⁶; in R¹, said "optionally substituted" means that the specified group is unsubstituted or is substituted by one or more R^{1s}, and each R^{1s} is independently selected from the group consisting of: halo, hydroxy, amino, cyano, oxo, C₁-C₆ alkyl, C₁-C₆ haloalkyl, C₁-C₆ alkoxy-C₁-C₆ alkyl, hydroxy-C₁-C₆ alkyl, C₁-C₆ alkylamino, C₁-C₆ alkoxy, and C₁-C₆ haloalkoxy.

3. The compound according to claim 1, or a pharmaceutically acceptable salt, enantiomer, diastereomer, tautomer, cis-trans isomer, solvate, polymorph, deuterated form thereof, or a combination thereof, **characterized in that**:
R² is selected from the group consisting of: halo, optionally substituted C₁-C₆ alkyl, optionally substituted 3- to 10-membered carbocyclyl, optionally substituted 4- to 8-membered heterocyclyl, and -L²-R¹⁰; in R², said "optionally substituted" means that the specified group is unsubstituted or is substituted by one or more R^{2s}, and each R^{2s} is independently selected from the group consisting of: halo, cyano, oxo, C₁-C₆ alkyl, C₁-C₆ haloalkyl, C₁-C₆ alkoxy, C₁-C₆ haloalkoxy, and 3- to 8-membered carbocyclyl;
R³ is selected from the group consisting of: hydrogen, halo, optionally substituted C₁-C₆ alkyl, optionally substituted C₂-C₆ alkenyl, optionally substituted C₂-C₆ alkynyl, optionally substituted 3- to 10-membered carbocyclyl, optionally substituted 4- to 8-membered heterocyclyl, -L^{2'}-R^{10'}; in R³, said "optionally substituted" means that the specified group is unsubstituted or is substituted by one or more R^{3s}, and each R^{3s} is independently selected from the group consisting of: halo, cyano, oxo, C₁-C₆ alkyl, C₁-C₆ haloalkyl, C₁-C₆ alkoxy, C₁-C₆ haloalkoxy, and 3- to 8-membered carbocyclyl;
R¹⁰ and R^{10'} are each independently selected from the group consisting of: optionally substituted C₁-C₆ alkyl, optionally substituted 3- to 8-membered carbocyclyl, optionally substituted 4- to 8-membered heterocyclyl; in R¹⁰ and R^{10'}, said "optionally substituted" means that the specified group is unsubstituted or is substituted by one or more R^{10s}, and each R^{10s} is independently selected from the group consisting of: halo, cyano, C₁-C₆ alkyl, C₁-C₆ haloalkyl, C₁-C₆ alkoxy, C₁-C₆ haloalkoxy, 3- to 8-membered carbocyclyl, 4- to 8-membered heterocyclyl.

4. The compound according to claim 1, or a pharmaceutically acceptable salt, enantiomer, diastereomer, tautomer, cis-trans isomer, solvate, polymorph, deuterated form thereof, or a combination thereof, **characterized in that**:
R^{X} is selected from the group consisting of: methyl, ethyl, halomethyl, and halo;
X¹ is selected from the group consisting of: CR¹³ and N;
X² is selected from the group consisting of: CR¹⁴ and N;
R¹³ and R¹⁴ are each independently selected from the group consisting of: hydrogen, halo, cyano, C₁-C₄ alkyl, or C₁-C₄ haloalkyl; with the proviso that when R^{X} and R² are both chloro and X¹ is CR¹³, R¹³ is not chloro.

5. The compound according to claim 1, or a pharmaceutically acceptable salt, enantiomer, diastereomer, tautomer, cis-trans isomer, solvate, polymorph, deuterated form thereof, or a combination thereof, **characterized in that**:
R^{X} is methyl or halo;
R^{Y} is hydrogen, methyl, or halo.

6. The compound according to claim 1, or a pharmaceutically acceptable salt, enantiomer, diastereomer, tautomer, cis-trans isomer, solvate, polymorph, deuterated form thereof, or a combination thereof, **characterized in that**:
R¹ is selected from the group consisting of: hydrogen, optionally substituted C₁-C₆ alkyl, and -NR⁵R⁶; wherein R⁵ is hydrogen, and R⁶ is selected from the group consisting of: hydrogen, optionally substituted C₁-C₆ alkyl, and optionally substituted 5- to 10-membered heteroaryl; in R⁶, said "optionally substituted" means that the specified group is unsubstituted or is substituted by one or more R^{6s}, and R^{6s} is deuterium or C₁-C₆ alkyl;
R² is selected from the group consisting of: halo, optionally substituted C₁-C₆ alkyl, and optionally substituted 3- to 10-membered carbocyclyl; in R², said "optionally substituted" is as set forth in claim 1;
R³ is hydrogen or -L^{2'}-R^{10'}; wherein L^{2'} is -O- and R^{10'} is methyl;
X¹ is CR¹³;
X² is CR¹⁴;
X³ is N;
R¹³ and R¹⁴ are each independently selected from the group consisting of: hydrogen, halo, cyano, C₁-C₄ alkyl, or C₁-C₄ haloalkyl;
R^{X} is methyl, halo, or cyclopropyl;
R^{Y} is hydrogen, methyl, or halo.

7. The compound according to any one of claims 1-6, or a pharmaceutically acceptable salt, enantiomer, diastereomer, tautomer, cis-trans isomer, solvate, polymorph, deuterated form thereof, or a combination thereof, **characterized in that** said compound is selected from Table A below:

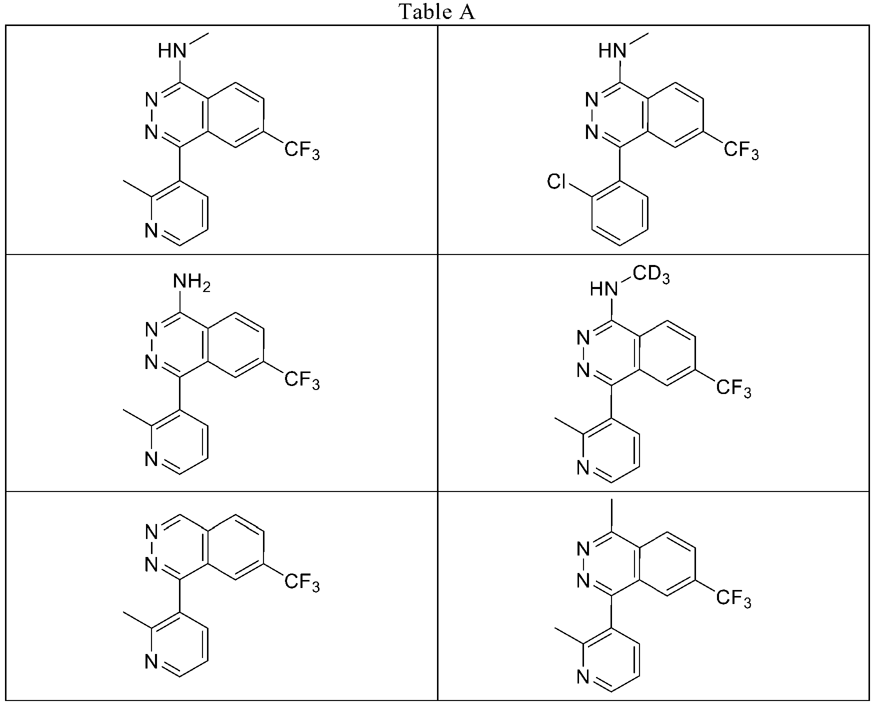

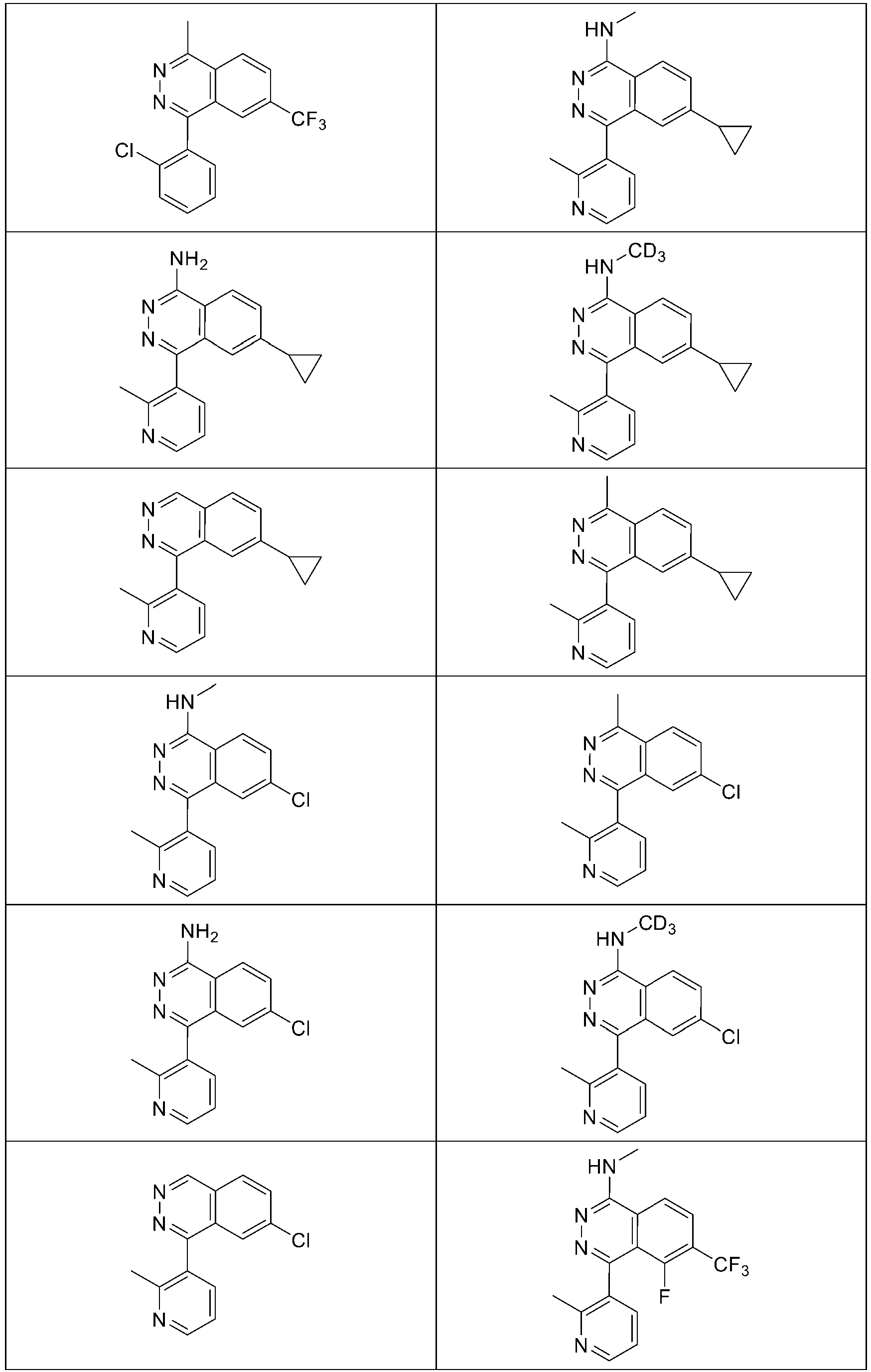

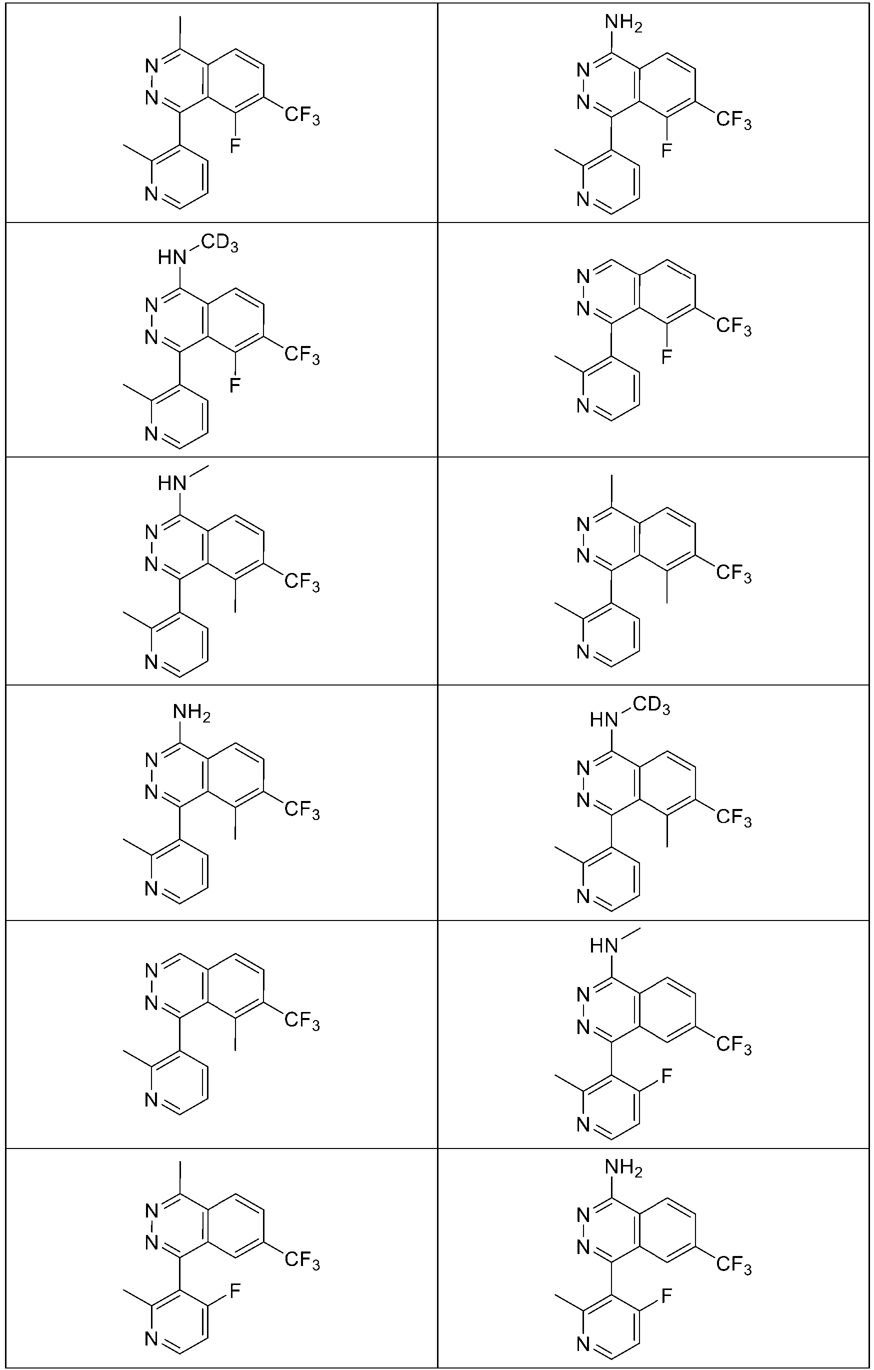

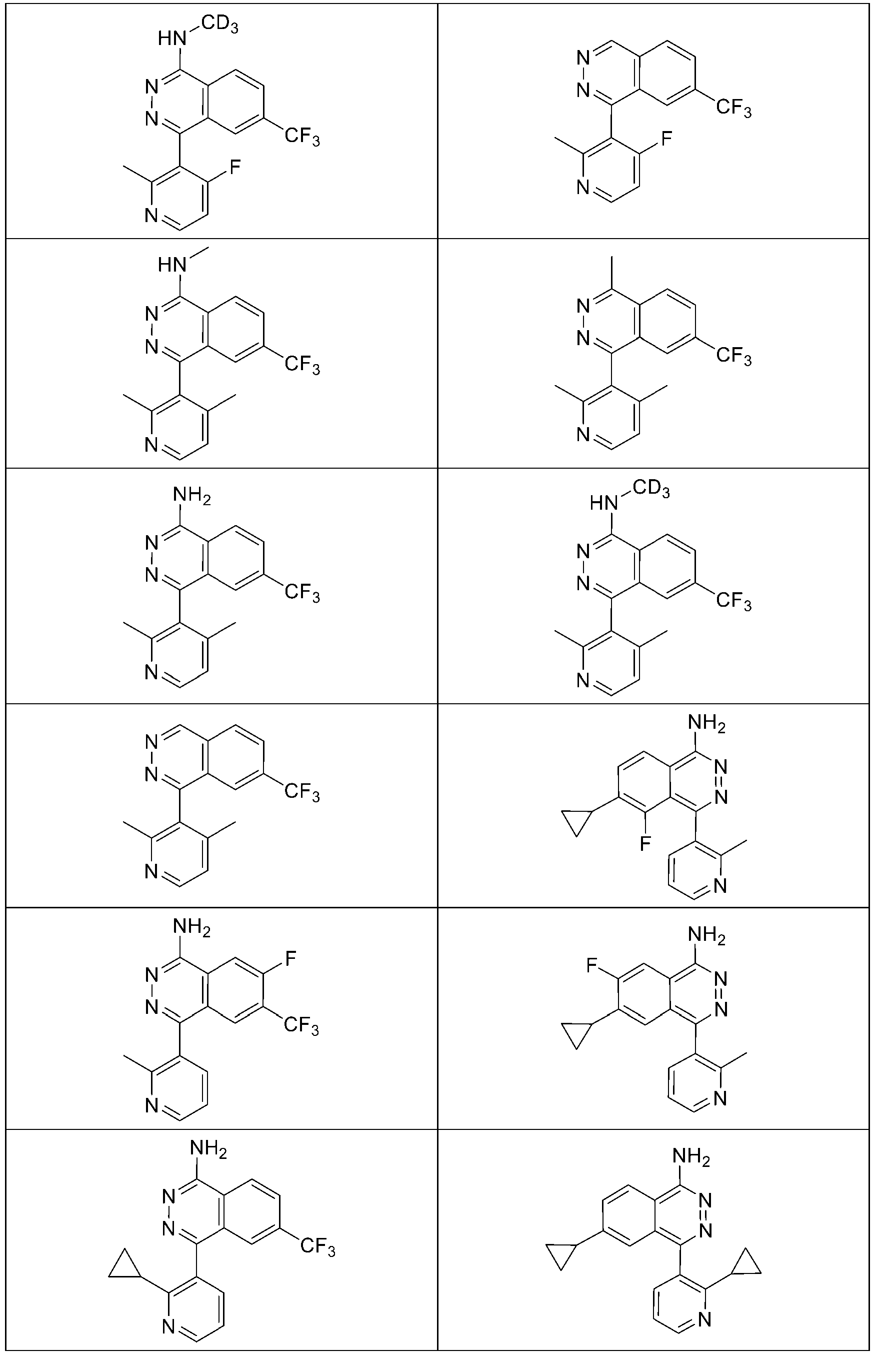

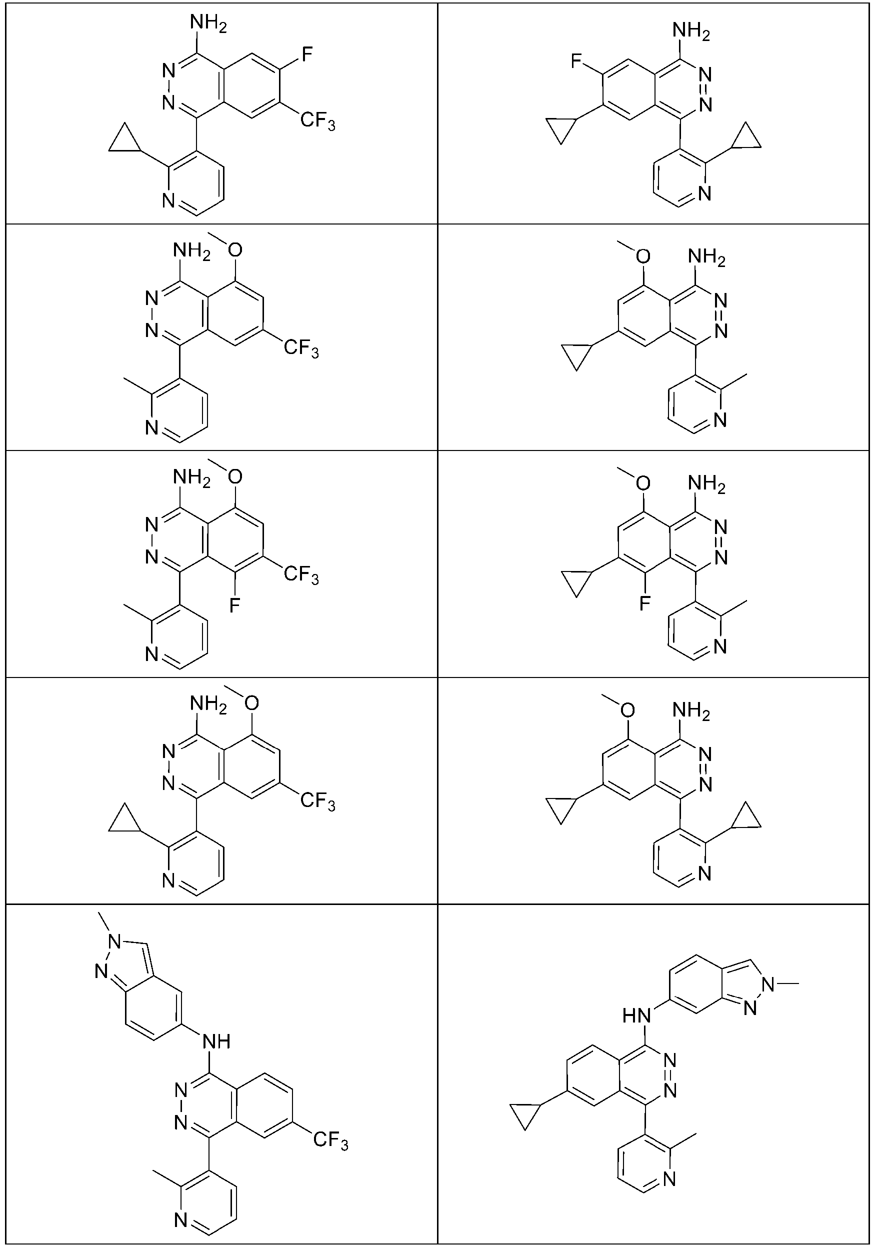

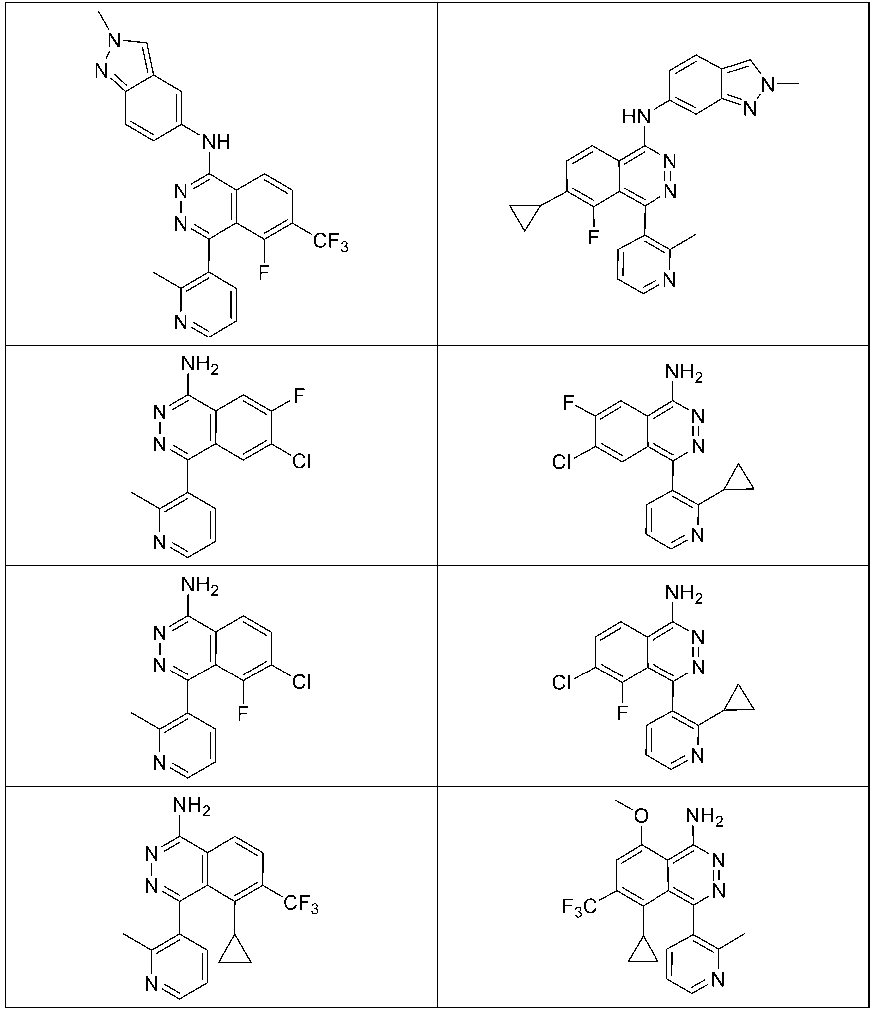

8. A pharmaceutical composition, **characterized in that** said pharmaceutical composition comprises:
(1) a therapeutically effective amount of the compound according to any one of claims 1 to 7, or one or more of a pharmaceutically acceptable salt, enantiomer, diastereomer, tautomer, cis-trans isomer, solvate, polymorph, and deuterated form thereof as the active ingredient; and
(2) optionally, a pharmaceutically acceptable carrier.

9. Use of the compound according to any one of claims 1-7, or a pharmaceutically acceptable salt, enantiomer, diastereomer, tautomer, cis-trans isomer, solvate, polymorph, or deuterated form thereof, or the pharmaceutical composition according to claim 8, in the preparation of a medicament for the prevention or treatment of MTAP^{-/-} associated cancers.

10. The use according to claim 9, **characterized in that** the cancer is selected from: glioma, mesothelioma, esophageal cancer, bladder cancer, urothelial carcinoma, pancreatic cancer, melanoma, non-small cell lung cancer, head and neck cancer, sarcoma, cholangiocarcinoma, liver cancer, breast cancer, skin cancer, intestinal cancer, gastric cancer, renal cancer, urethral cancer, prostate cancer, testicular cancer, uterine cancer, ovarian cancer, vaginal cancer, fallopian tube cancer, multiple myeloma, spinal neurofibroma, astrocytoma, acute lymphoblastic leukemia, chronic lymphocytic leukemia, Hodgkin lymphoma, and non-Hodgkin lymphoma.
